# EUROPEAN PATENT APPLICATION

(11) **EP 4 678 194 A1**
(43) Date of publication of application: **14.01.2026**
(21) Application number: 24188412.1
(22) Date of filing: 12.07.2024
(51) Int. Cl.: A61K 51/08, A61P 35/00, A61K 103/00

(54) **THERAPEUTIC APPLICATION OF 64CU-LABELLED CONJUGATES FOR SITE-SPECIFIC UPAR-TARGETING**

(71) Applicant: Curasight A/S, 2200 Copenhagen N (DK)
(72) Inventor: Kjær, Andreas, 2000 Frederiksberg (DK)
(74) Representative: Budde Schou A/S

(57) **Abstract**

The present invention provides Therapeutic application of 64Cu labelled uPAR binding conjugates, such as 64Cu-DOTA-AE105, for site-specific targeting of the Urokinase Plasminogen Activator Receptor (uPAR) in treatment of cancer diseases associated with high uPAR expression.

## Description

### FIELD OF THE INVENTION

The present invention relates to a radionuclide labelled conjugates for site-specific targeting of the Urokinase Plasminogen Activator Receptor (uPAR) in particular the treatment of cancer diseases associated with high uPAR expression.

### BACKGROUND OF THE INVENTION

Various radionuclide labelled targeting moieties, such as peptide compositions, have been developed or are under development for site-specific targeting of a therapeutic radionuclide. The general principle involves attaching a selected radionuclide to a targeting moiety, such as a peptide, having a high specificity for a particular organ or tissue so that the organ or tissue can be treated by a therapeutic radioisotope. This field of research has shown promising applicability for tumor treatment.

Therapeutic radionuclides are radioactive isotopes having a set of properties that will deliver targeted radiation to diseased tissues, with minimal damage to surrounding healthy tissues. In order to achieve localized radiation a key feature of a therapeutic radionuclide is the type of radiation emitted. Therapeutic radionuclides emit high-linear energy transfer (LET) radiation typically in the form of beta minus (β-) particles or alpha minus (α-) particles providing a high ionization per length of travel, which is fully deposited within a small range of tissue, usually in the range of mm, thereby minimizing damage of surrounding healthy tissue. Beta minus emitters, such as Yttrium-90 (90Y), Copper-67 (67Cu), and Lutetium-177 (177Lu), are commonly used due to their moderate tissue penetration and suitable half-lives. Alpha emitters, like Actinium-225 (225Ac) and Radium-223 (223Ra), provide high linear energy transfer (LET), which results in significant cytotoxicity within a short range, making them ideal for targeting small clusters of cancer cells.

Furthermore, relevant features for the selection of an appropriate radionuclide for therapy also includes the radionuclide half-life and ability to be conjugated to targeting molecules, such as peptides.

The half-life of a radionuclide influences its clinical applicability. It must be long enough to allow for manufacturing, quality control, and administration, yet short enough to minimize unnecessary radiation exposure to the patient. For example, Yttrium-90 (90Y) has a half-life of 64.1 hours, Copper-67 (67Cu) has a half-life of 2.6 days, Lutetium-177 (177Lu) has a half-life of 6.7 days, Radium-223 (223Ra) has a half-life of 11.4 days, and Actinium-225 (225Ac) has a half-life of 10 days.

Radionuclides used in medical imaging possess distinct physical and chemical properties that make them suitable for diagnostic purposes. Their efficacy in imaging relies on several key characteristics, including half-life, type of emitted radiation, energy of radiation, and chemical compatibility with biological systems. The half-life of a radionuclide is critical in imaging as it determines the duration of its activity within the body. Ideal radionuclides for diagnostic imaging have a half-life that is long enough to perform the imaging procedure but short enough to minimize radiation exposure to the patient. For imaging purposes, gamma-emitting radionuclides are primarily used due to their ability to penetrate tissues and be detected by gamma cameras. Positron emitters are also employed, particularly in Positron Emission Tomography (PET) imaging, where the positron annihilates with an electron, producing two gamma photons that are detected simultaneously.

The radionuclide 64Cu is a well-known positron emitting radionuclide with a half-life of 12.7 hours and has been used for labeling imaging agents successfully. In addition to positron emission, 64Cu also decays by both beta minus particles and gamma radiation. Organ dosimetry calculations based on the human biodistribution of labelled compounds have, however, deemed 64Cu ineffective for therapeutic purposes. For this reason, 64Cu labelled compounds are used as imaging agents either alone for diagnostic purposes or combined with a 67Cu labelled counterpart for therapeutic purposes working together as a theranostic pair. One example may be 64Cu-SARTATE and 67Cu-SARTATE developed by Clarity pharmaceuticals.

Malignant tumors are capable of degrading the surrounding extracellular matrix, resulting in local invasion or metastasis. Urokinase-type plasminogen activator (uPA) and its cell surface receptor (uPAR) are central molecules for cell surface-associated plasminogen activation both in vitro and in vivo. uPAR is overexpressed in a variety of human cancers whereas the expression in non-cancer tissue is low and correlates with malignant tumor growth and is associate with a poor prognosis in many types of human cancers, possibly indicating a causal role for the uPA/uPAR system in cancer progression and metastasis. Studies by immunohistochemistry and in situ hybridization indicate that expression levels of the components from the uPA system are generally very low in normal tissues and benign lesions. It has also been reported that the uPA/uPAR system is involved in regulating cell-extracellular matrix interactions by acting as an adhesion receptor for vitronectin and by modulating integrin function. Based on these properties the uPA/uPAR system is consequently considered an attractive target for cancer therapy, and uPAR binding peptides including the peptide AE105 have been developed for targeting of the uPA/uPAR system such as described in US 7,026,282. Furthermore, the radionuclide labelled uPAR-targeting peptide conjugate 177Lu-DOTA-AE105 has been developed in the pursue of providing targeted radiotherapy for uPAR expressing cancers as described in WO 2013/167130. However, the tumor binding, as well as tumor retention of this peptide conjugate, was vastly decreased compared to that of the peptide alone, indicating that the AE105 peptide is not well suited for labeling with therapeutic radionuclide and has limited capabilities for providing targeted radiotherapy.

Development of therapeutic radionuclide labelled peptide conjugates is a complex process posing various challenges such as the stability of the conjugate, which can be challenged by e.g. radiolysis by the therapeutic radionuclides and insufficient binding of the radionuclide within the chelator in a given conjugate structure. Furthermore, and particularly for therapeutic applications, target specificity is critical to achieve low toxicity as well as a therapeutic effect. Also, retention of the conjugate at target is important for achieving optimal therapeutic effect. Despite extensive research pursuing peptide conjugates targeting various molecules, only two such conjugates have at present been approved for therapy, that is 177Lu-DOTA-TATE (Lutathera) and 177Lu-DOTA-PSMA (Pluvicto) for treatment of gastroenteropancreatic neuroendocrine tumors (GEP-NETs), which are positive for the hormone receptor somatostatin and metastatic castration-resistant prostate cancer (PSMA-positive mCRPC), respectively.

Thus, there is a need for development of stable radionuclide labelled uPAR-targeting conjugates providing sufficient tumor binding and good tumor retention, so that cancer diseases associated with high uPAR expression can be treated with targeted radiotherapy.

### SUMMARY OF THE INVENTION

It was surprisingly found that the radionuclide 64Cu well known for its imaging properties provides therapeutic efficacy at doses far below the theoretical dose, thereby enabling therapeutic application of 64Cu labeled uPAR binding conjugates according to the present invention in human patients suffering from uPAR expressing cancers.

In a first aspect, the present invention relates to a radionuclide labelled uPAR binding conjugate for use in treatment of uPAR expressing cancer, wherein said radionuclide labelled uPAR conjugate comprises:
a) A uPAR binding moiety
b) A chelating agent suitable for binding radiometals
c) The radionuclide 64Cu
And wherein the peptide is coupled to 64Cu by the chelating agent.

Preferably the radionuclide labelled uPAR binding conjugate for use according to the first aspect, wherein said uPAR binding moiety is a uPAR binding peptide.

In a second aspect, the present invention relates to a method of treatment of a uPAR expressing cancer disease by administering to a patient a radionuclide labeled uPAR binding conjugate, wherein sad radionuclide labeled uPAR binding conjugate comprises:
a) A uPAR binding moiety
b) A chelating agent suitable for binding radiometals
c) The radionuclide 64Cu
And wherein the peptide is coupled to 64Cu by the chelating agent.

Preferably, the method of treatment according to the second aspect, wherein said uPAR binding moiety is a uPAR binding peptide.

In a third aspect, the present invention relates to a pharmaceutical formulation comprising a radionuclide labeled uPAR binding peptide conjugate comprising:
a) A uPAR binding moiety, preferably a uPAR binding peptide
b) A chelating agent suitable for binding radiometals
c) The radionuclide 64Cu
And wherein the peptide is coupled to 64Cu by the chelating agent.

The 64Cu labelled peptide conjugates of the present invention having a high tumor binding and good tumor retention, offers a much wider therapeutic window in which the dose providing the optimal balance of therapeutic effect and side effects can be achieved compared to inferior counterparts.

For the purpose of completeness, it is herewith clarified that the radionuclide labelled uPAR binding conjugates according to the invention and all the embodiments are preferably consisting of the specified components. That is, in a preferred form, the present invention relates to:
a radionuclide labelled uPAR binding conjugate for use in treatment of uPAR expressing cancer, wherein said radionuclide labelled uPAR conjugate consists of
   a) A uPAR binding moiety, preferably a uPAR binding peptide
   b) A chelating agent suitable for binding radiometals
   c) The radionuclide 64Cu
And wherein the peptide is coupled to 64Cu by the chelating agent.
And,

A method of treatment of a uPAR expressing cancer disease by administering to a patient a radionuclide labeled uPAR binding conjugate, wherein said radionuclide labeled uPAR binding conjugate consists of:
a) A uPAR binding moiety, preferably a uPAR binding peptide
b) A chelating agent suitable for binding radiometals
c) The radionuclide 64Cu
And wherein the peptide is coupled to 64Cu by the chelating agent.
And,

A pharmaceutical formulation comprising a radionuclide labeled uPAR binding conjugate consisting of:
a) A uPAR binding moiety, preferably a uPAR binding peptide
b) A chelating agent suitable for binding radiometals
c) The radionuclide 64Cu
And wherein the peptide is coupled to 64Cu by the chelating agent.

The 64Cu labelled conjugates of the present invention, in particular 64Cu labelled peptide conjugates, having a high therapeutic effect at relatively low doses, a high tumor binding, and good tumor retention, offers a much wider therapeutic window in which the dose providing the optimal balance of therapeutic effect and side effects can be achieved compared to inferior counterparts.

### BRIEF DESCRIPTION OF THE DRAWINGS

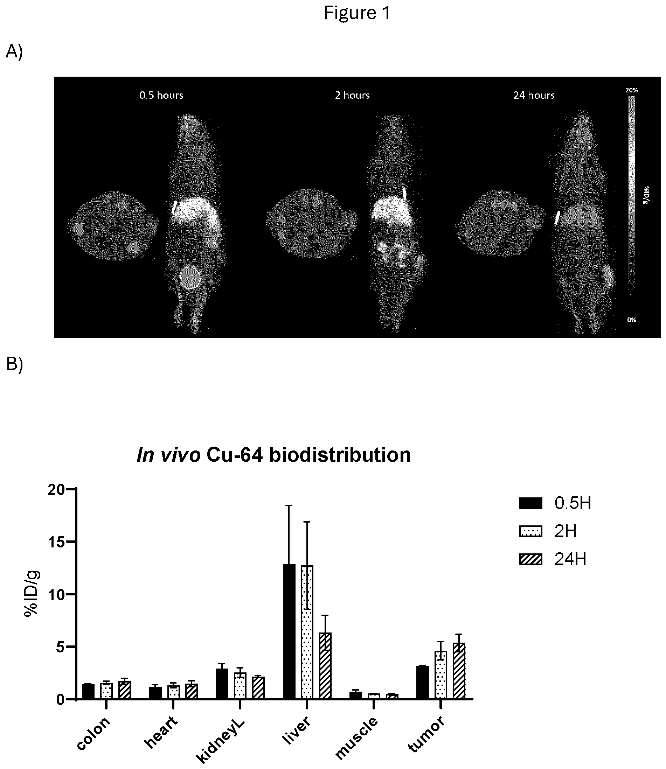

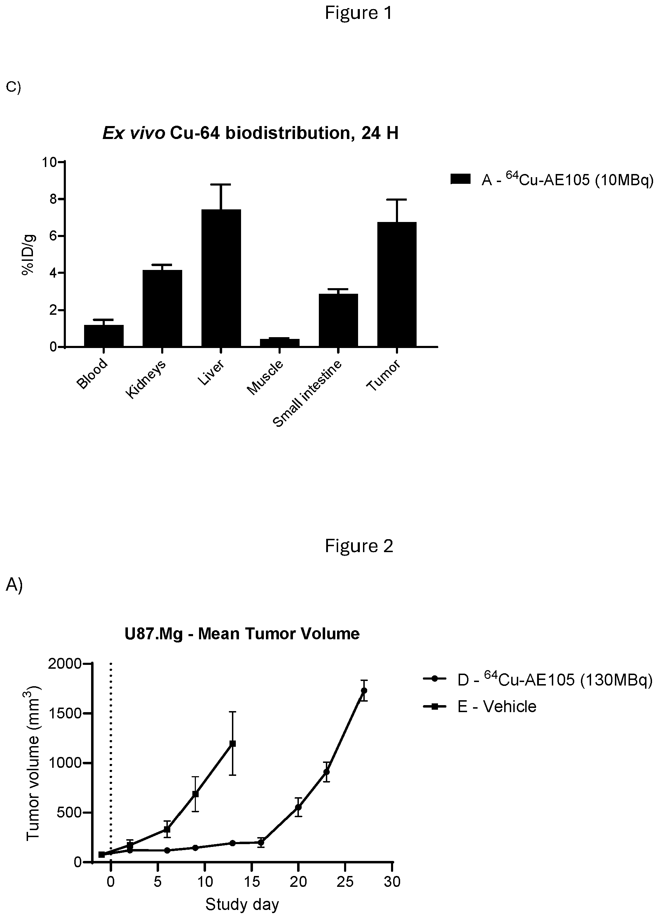

**Figure 1****:** A) shows a representative PET/CT images of the biodistribution of 64Cu-DOTA-AE105 in mice with xenograft tumors (U87MG). Images are taken 0.5H, 2H, and 24H after injection of 10 MBq Cu-64-DOTA-AE105. For each timepoint, the left image represents an axial slice intersecting the center of the tumor. The right image represents the MIP image. B) shows *In vivo* biodistribution 0.5H, 2H and 24H post-dosing of 10 MBq Cu-64-DOTA-AE105, estimated by quantitative region interest (ROI) analysis. Graph show %ID/g for all listed organs. N=2-3. Bars represent mean ± SEM. C) shows *Ex vivo* biodistribution of Cu-64-DOTA-AE105 24H post-dosing of 10 MBq Cu-64DOTA-AE105. Bars represent mean ± SEM. N=3.

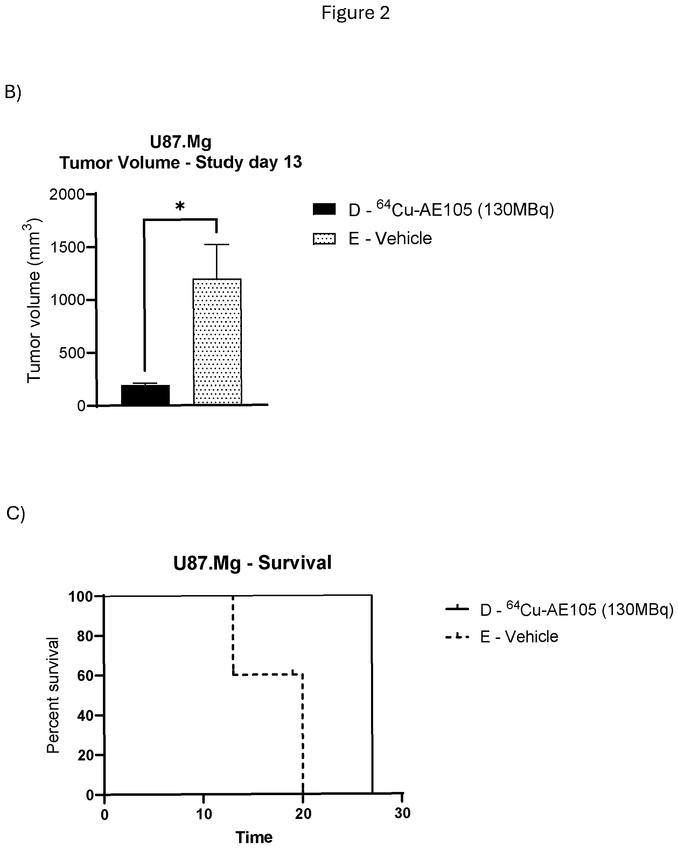

**Figure 2****:** A) shows mean tumor growth for up to 3 weeks after administration of a single dose of 130 MBq of 64Cu-DOTA-AE105 as therapy. Dose was administered at day 0. Carry forward was applied for mean tumor growth curve until <60% animals were still alive in the groups. The vertical dotted lines indicate the initiation of treatment with Cu-64-DOTA-AE105. Bars represent mean ± SEM. N=5/group. B) shows tumor volume comparison at study day 13 (the last day where 60% or more animals were alive within the two groups). The effect of Cu-64-DOTA-AE105 was tested against Vehicle. A significant effect was observed following treatment with Cu-64-DOTA-AE105 compared to Vehicle (Unpaired t-test, p=0.0139). Bars represent mean ± SEM. N=5/group. C) shows animal survival for up to 27 days after treatment initiation. Each event represents an animal euthanized to humane endpoints. Animals euthanized due to tumor wounds are censured in the survival curve. A significant survival was observed following treatment with Cu-64-DOTA-AE105 compared to Vehicle (Mantel Cox's test, P= 0.0164). N= 5/group.

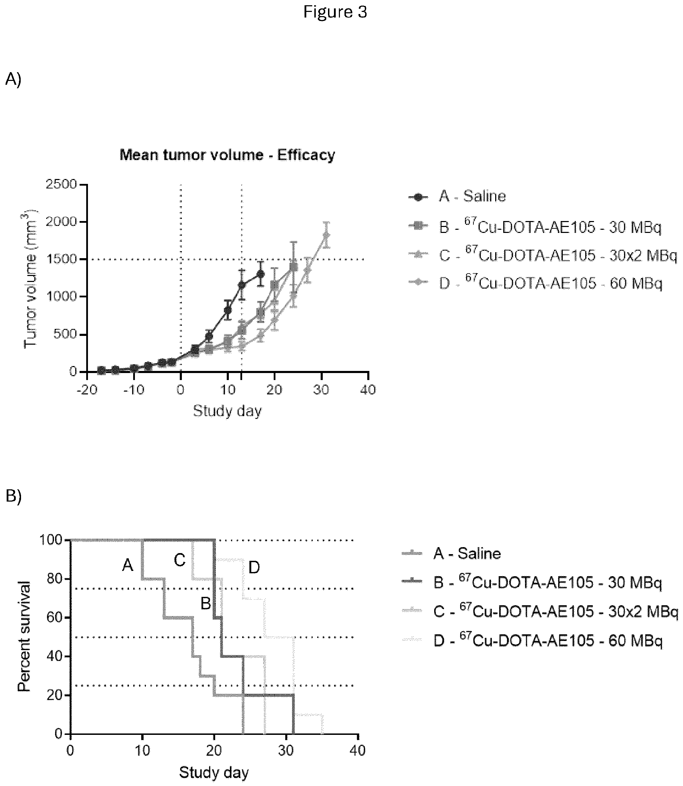

**Figure 3:** A) shows treatment efficacy of 67Cu-DOTA-AE105 in a human glioblastoma xenograft mouse model. B) shows Kaplan-Meier curves of survival data from the treatment efficacy study of 67Cu-DOTA-AE105 in a human glioblastoma xenograft mouse model.

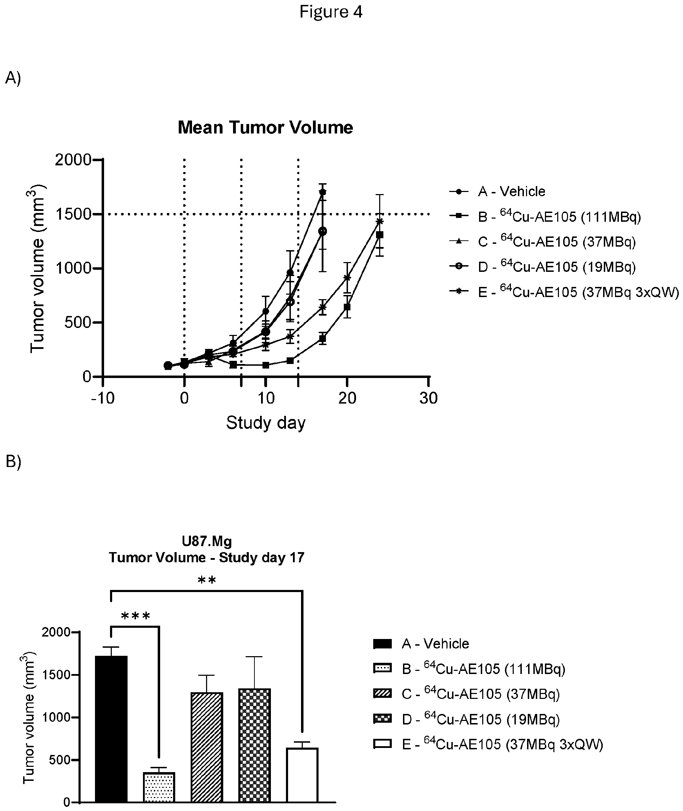

**Figure 4:** A) shows mean tumor growth of group A-E for up to 5 weeks after therapy initiation. Carry forward was applied for mean tumor growth curve until <60% animals were still alive in the groups. The vertical dotted lines indicate the initiation of treatment with Cu-64-DOTA-AE105. Bars represent mean ± SEM. N=5/group. B) shows tumor volume comparison at study day 17 (the last day where 60% or more animals were alive within all the groups). The effect of a single dose of 19, 37 and 111 MBq Cu-64-DOTA-AE105 (Group B-D) and the effect of fractionated dosing of 3 times 37 MBq Cu-64-DOTA-AE105 (Group E) were tested against Vehicle (Group A) by One-way ANOVA with Dunnett's multiple comparison. A significant effect was observed following treatment with 111MBq Cu-64-DOTA-AE105 compared to Vehicle and 3 times 37 MBq Cu-64-DOTA-AE105 as fractionated dosing. Bars represent mean ± SEM. N=3-5/group.

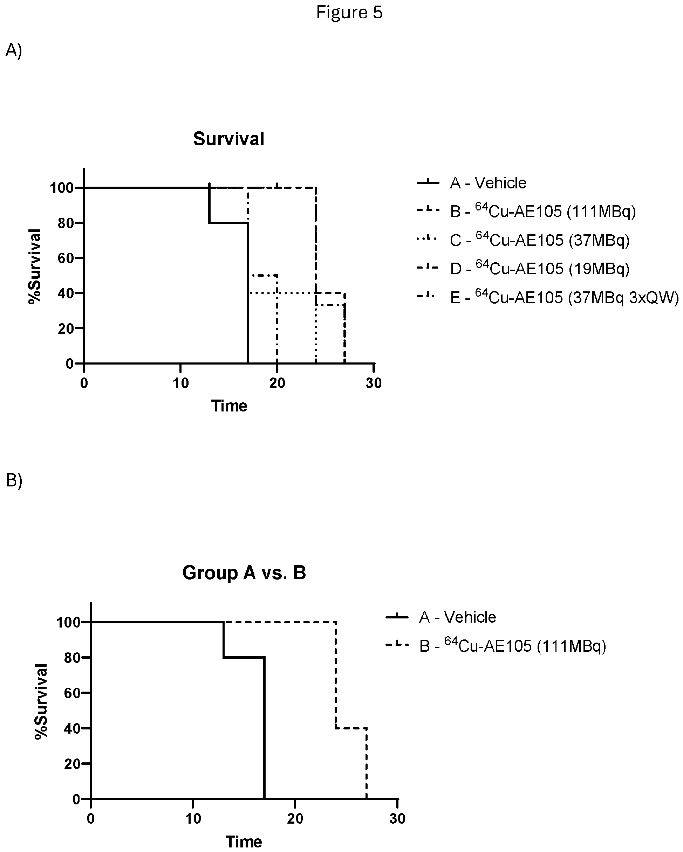

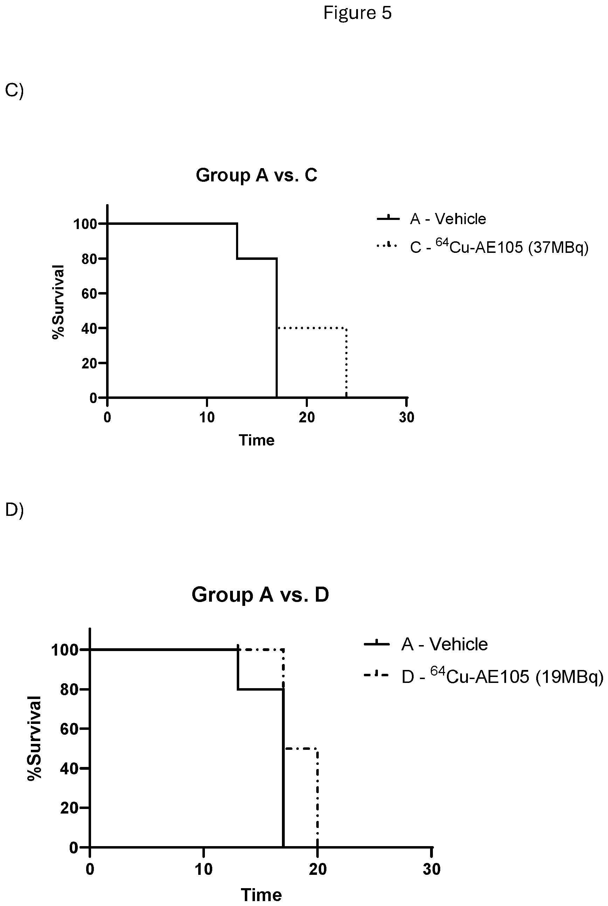

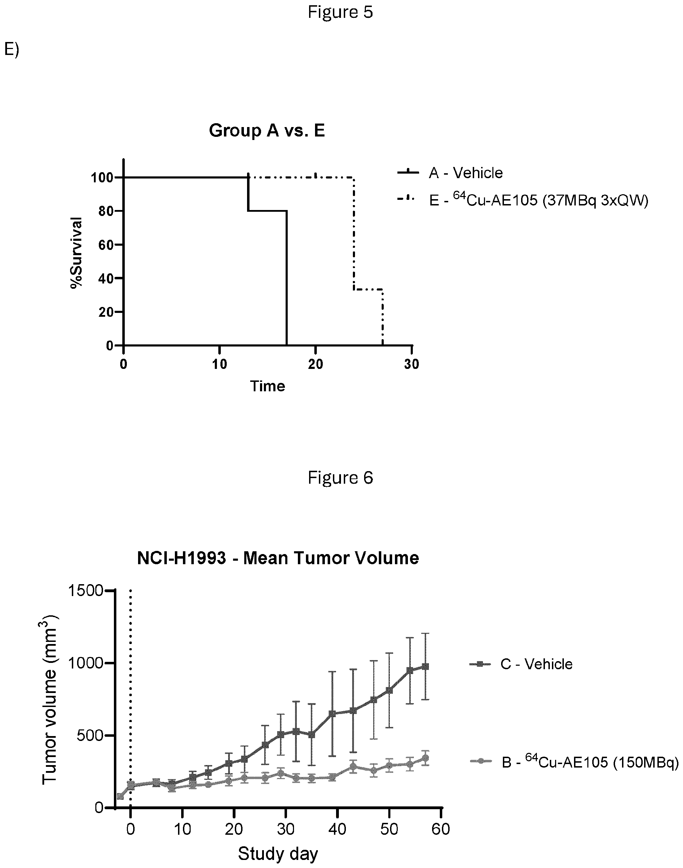

**Figure 5:** A) to E) show animal survival for groups A-E for up to 5 weeks after treatment initiation. Each event represents an animal euthanized to humane endpoints. Animals euthanized due to tumor wounds are censured in the survival curve. N= 5/group.
**Figure 6****:** shows mean tumor growth for up to 8 weeks after administration of a single dose of 150MBq of 64Cu-DOTA-AE105 in a NCI-H1993 tumor model. Carry forward was applied for mean tumor growth curve until <60% animals were still alive in the groups. The vertical dotted lines indicate the initiation of treatment with Cu-64-DOTA-AE105. Bars represent mean ± SEM. N=5/group.

### DETAILED DESCRIPTION OF THE INVENTION

It was surprisingly found that the radionuclide 64Cu well known for its imaging properties provides therapeutic efficacy at doses far below the theoretical dose thereby enabling therapeutic application of 64Cu labeled uPAR binding conjugates according to the present invention, such as 64Cu labeled uPAR binding peptide conjugates, in human patients suffering from uPAR expressing cancers.

The **becquerel** (Bq) is the unit of radioactivity in the International System of Units (SI). One becquerel is defined as an activity of one decay per second. Bq is a measure of activity dose (what is administered). In context of human dosing, the commonly used multiples M and G are used, i.e. MBq (megabecquerel, 10⁶ Bq) GBq (gigabecquerel, 10⁹ Bq).

The **gray** (**Gy**) is the unit of ionizing radiation dose in the International System of Units (SI), defined as the absorption of one joule of radiation energy per kilogram of matter. Thus, Gy is a measure of deposited dose in tissue.

**OLINDA/EXM** is the most widely used and FDA approved software for standardized dose calculations for diagnostic and therapeutic radiopharmaceuticals (Stabin MG, Sparks RB, Crowe E. OLINDA/EXM: the second-generation personal computer software for internal dose assessment in nuclear medicine. J Nucl Med. 2005 Jun;46(6):1023-7. PMID: 15937315. Stabin MG et al. 2005, and FDA.gov 2004 FDA approval of Olinda Exm (https://www.accessdata.fda.gov/cdrh_docs/pdf3/k033960.pdf)). The program calculates the absorbed radiation dose to various organs according to the MIRD (Medical Internal Radiation Dose Committee of the Society of Nuclear Medicine) technique.

Based on the human biodistribution of the imaging ligand 64Cu-DOTA-AE105, , the inventor calculated the organ dosimetry to be anticipated from 67Cu-DOTA-AE105 (table 1), using the standard software OLINDA/EXM.

**Table 1: Organ doses of 67Cu-DOTA-AE105 are given in mGy/MBq. Effective dose is given in units of mSv/MBq.**

| **Target Organ** | **Alpha** | **Beta** | **Gamma** | **Total** |
|---|---|---|---|---|
| Adrenals | 0,00E+00 | 1,19E-01 | 7,99E-02 | 1,99E-01 |
| Brain | 0,00E+00 | 5,69E-03 | 4,93E-03 | 1,06E-02 |
| Esophagus | 0,00E+00 | 3,46E-02 | 3,76E-02 | 7,22E-02 |
| Eyes | 0,00E+00 | 3,45E-02 | 8,50E-03 | 4,30E-02 |
| Gallbladder Wall | 0,00E+00 | 2,14E-01 | 1,19E-01 | 3,33E-01 |
| Left colon | 0,00E+00 | 1,13E-01 | 3,11E-02 | 1,44E-01 |
| Small Intestine | 0,00E+00 | 1,29E-01 | 3,25E-02 | 1,62E-01 |
| Stomach Wall | 0,00E+00 | 8,21E-02 | 3,93E-02 | 1,21E-01 |
| Right colon | 0,00E+00 | 1,74E-01 | 4,92E-02 | 2,24E-01 |
| Rectum | 0,00E+00 | 3,45E-02 | 1,87E-02 | 5,32E-02 |
| Heart Wall | 0,00E+00 | 9,61E-02 | 4,27E-02 | 1,39E-01 |
| Kidneys | 0,00E+00 | 2,42E-01 | 5,50E-02 | 2,97E-01 |
| Liver | 0,00E+00 | 1,01E+00 | 1,48E-01 | 1,16E+00 |
| Lungs | 0,00E+00 | 2,25E-02 | 3,05E-02 | 5,30E-02 |
| Pancreas | 0,00E+00 | 2,33E-01 | 5,95E-02 | 2,93E-01 |
| Prostate | 0,00E+00 | 3,45E-02 | 1,88E-02 | 5,34E-02 |
| Salivary Glands | 0,00E+00 | 3,45E-02 | 1,20E-02 | 4,65E-02 |
| Red Marrow | 0,00E+00 | 6,28E-02 | 1,99E-02 | 8,28E-02 |
| Osteogenic Cells | 0,00E+00 | 1,01E-01 | 2,98E-02 | 1,31E-01 |
| Spleen | 0,00E+00 | 7,70E-02 | 2,65E-02 | 1,04E-01 |
| Testes | 0,00E+00 | 3,45E-02 | 1,12 E-02 | 4,58E-02 |
| Thymus | 0,00E+00 | 3,46E-02 | 2,27E-02 | 5,73E-02 |
| Thyroid | 0,00E+00 | 6,44E-02 | 1,63E-02 | 8,07E-02 |
| Urinary Bladder Wall | 0,00E+00 | 7,54E-02 | 1,87E-02 | 9,41E-02 |
| Total Body | 0,00E+00 | 6,70E-02 | 1,58E-02 | 8,28E-02 |
| Effective Dose | 1,25E-01 | | | |

Combining these calculations with the generally accepted maximum allowed radiation doses to organs (Wahl (Wahl et al., 2021) and Emami (Emami, 2013)) and table 2, central column, maximal allowed administered doses of 67Cu-DOTA-AE105 for each organ could be calculated (table 2, right column) and the dose limiting organ was red bone marrow with a limit of administered radioactivity dose of 24.2 GBq 67Cu-DOTA-AE105.

**Table 2: Calculated values for maximum allowed activity of 67Cu-DOTA-AE105. Values are based on dose limits from Wahl et al. 2021 and Emami et al. 2015.**

| **Organ** | **Allowed Dose (Gy)** | **Maximum allowed Activity (GBq)** |
|---|---|---|
| Left colon | 25 | 1,74E+02 |
| small intestine | 18 | 1,11E+02 |
| stomach wall | 28 | 2,31E+02 |
| right colon | 25 | 1,12E+02 |
| rectum | 25 | 4,70E+02 |
| heart wall | 32 | 2,30E+02 |
| kidneys | 23 | 7,74E+01 |
| liver | 30 | 2,59E+01 |
| lungs | 12 | 2,26E+02 |
| urinary bladder wall | 65 | 6,91E+02 |
| **Red marrow** | **2** | **2,42E+01** |

Assuming a tumor uptake of a Standardized Uptake Value (SUV) of 10, which is a relevant level for tumors in general with our compound (Persson et al 2015), would result in a radiation dose to a 10 g tumor of 26.1 Gy (for dependence of tumor size, please see table 3).

**Table 3: Calculated total tumor dose at maximum administered activity, as a function of spherical tumor size.**

| **Sphere mass (g)** | **Gy** |
|---|---|
| 0.01 | 2,37E+01 |
| 0.1 | 2,48E+01 |
| 0.5 | 2,51E+01 |
| 1 | 2,55E+01 |
| 2 | 2,57E+01 |
| 4 | 2,58E+01 |
| 6 | 2,59E+01 |
| 8 | 2,60E+01 |
| 10 | 2,61E+01 |
| 20 | 2,63E+01 |
| 40 | 2,65E+01 |
| 60 | 2,66E+01 |
| 80 | 2,67E+01 |
| 100 | 2,68E+01 |

Generally accepted radiation doses needed for palliative radiation therapy are >30 Gy and for curative radiation therapy >50 Gy. Accordingly, the target dose to tumors cannot be achieved before reaching the dose limit of healthy organs.

When the same calculations are made for the imaging ligand 64Cu-DOTA-AE105, a maximal administered dose of 112 GBq of 64Cu-DOTA-AE105 was calculated to be the highest dose before reaching the maximum dose in the dose-limiting organ (red bone marrow). At this dose, the radiation dose delivered to a 10g tumor with an uptake of SUV=10, would then be 20.8 Gy leading to the same conclusion that the target doses of >30 Gy (palliative treatment) or >50 Gy (curative treatment) could not be achieved before reaching the dose limit of healthy organs. For details, please see tables 4, 5, and 6.

**Table 4: Organ doses of 64Cu-DOTA-AE105 are given in mGy/MBq. Effective dose is given in units of mSv/MBq.**

| **Target Organ** | **Alpha** | **Beta** | **Gamma** | **Total** |
|---|---|---|---|---|
| Adrenals | 0,00E+00 | 2,15E-02 | 2,21E-02 | 4,36E-02 |
| Brain | 0,00E+00 | 9,95E-04 | 1,80E-03 | 2,80E-03 |
| Esophagus | 0.00E+00 | 7,09E-03 | 1,01E-02 | 1,71E-02 |
| Eyes | 0,00E+00 | 7,08E-03 | 3,22E-03 | 1,03E-02 |
| Gallbladder Wall | 0,00E+00 | 2,89E-02 | 3,10E-02 | 5,99E-02 |
| Left colon | 0,00E+00 | 1,68E-02 | 9,71E-03 | 2,65E-02 |
| Small Intestine | 0,00E+00 | 2,07E-02 | 1,02E-02 | 3,09E-02 |
| Stomach Wall | 0,00E+00 | 1,37E-02 | 1,16E-02 | 2,53E-02 |
| Right colon | 0,00E+00 | 2,32E-02 | 1,36E-02 | 3,68E-02 |
| Rectum | 0,00E+00 | 7,08E-03 | 7,28E-03 | 1,44E-02 |
| Heart Wall | 0,00E+00 | 2,17E-02 | 1,28E-02 | 3,45E-02 |
| Kidneys | 0,00E+00 | 4,86E-02 | 1,68E-02 | 6,54E-02 |
| Liver | 0,00E+00 | 1,44E-01 | 3,98E-02 | 1,84E-01 |
| Lungs | 0,00E+00 | 4,71E-03 | 8,80E-03 | 1,35E-02 |
| Pancreas | 0,00E+00 | 4,58E-02 | 1,76E-02 | 6,33E-02 |
| Prostate | 0.00E+00 | 7,08E-03 | 7,73E-03 | 1,48E-02 |
| Salivary Glands | 0,00E+00 | 7,08E-03 | 4,47E-03 | 1,16E-02 |
| Red Marrow | 0,00E+00 | 1,10E-02 | 6,91E-03 | 1,79E-02 |
| Osteogenic Cells | 0.00E+00 | 1,73E-02 | 6,07E-03 | 2,34E-02 |
| Spleen | 0,00E+00 | 1,40E-02 | 8,66E-03 | 2,26E-02 |
| Testes | 0.00E+00 | 7,08E-03 | 4,74E-03 | 1,18E-02 |
| Thymus | 0.00E+00 | 7,12E-03 | 7,18E-03 | 1,43E-02 |
| Thyroid | 0,00E+00 | 1,39E-02 | 5,76E-03 | 1,96E-02 |
| Urinary Bladder Wall | 0,00E+00 | 4,10E-02 | 1,13E-02 | 5,23E-02 |
| Total Body | 0,00E+00 | 1,22E-02 | 5,48E-03 | 1,76E-02 |
| Effective Dose | 2,51E-02 | | | |

**Table 5: Calculated values for maximum allowed activity of 64Cu-DOTA-AE105. Values are based on dose limits from Wahl et al 2021 and Emami et al. 2015.**

| **Organ** | **Allowed Dose (Gy)** | **Maximum allowed Activity (GBq)** |
|---|---|---|
| Left colon | 25 | 9,43E+02 |
| small intestine | 18 | 5,83E+02 |
| stomach wall | 28 | 1,11E+03 |
| right colon | 25 | 6,79E+02 |
| rectum | 25 | 1,74E+03 |
| heart wall | 32 | 9,28E+02 |
| kidneys | 23 | 3,52E+02 |
| liver | 30 | 1,63E+02 |
| lungs | 12 | 8,89E+02 |
| urinary bladder wall | 65 | 1,24E+03 |
| **Red marrow** | **2** | **1,12E+02** |

**Table 6: Calculated total tumor dose at maximum administered activity, as a function of spherical tumor size.**

| **Sphere mass (g)** | **Gy** |
|---|---|
| 0.01 | 1,70E+01 |
| 0.1 | 1,88E+01 |
| 0.5 | 1,94E+01 |
| 1 | 2.00E+01 |
| 2 | 2,03E+01 |
| 4 | 2,05E+01 |
| 6 | 2,06E+01 |
| 8 | 2,07E+01 |
| 10 | 2,08E+01 |
| 20 | 2,12E+01 |
| 40 | 2,15E+01 |
| 60 | 2,17E+01 |
| 80 | 2,20E+01 |
| 100 | 2,21E+01 |

From the calculations using OLINDA/EXM software, it can also be concluded that a 5-6 fold higher administered activity dose of 64Cu-DOTA-AE105 compared to 67Cu-DOTA-AE105 is needed to obtain the same radiation dose to a tumor (112 GBq/20.8 Gy = 5.4 GBq/Gy for 64Cu-DOTA-AE105 vs. 24.2 GBq/26.1 Gy = 0.93 GBq/Gy for 67Cu-DOTA-AE105).

However, surprisingly, the inventor found in a series of experiments that this was not the case. Accordingly, in comparative tumor efficacy studies using either 67Cu-DOTA-AE105 or 64Cu-DOTA-AE105 it was found that a dose of 111 MBq of 64Cu-DOTA-AE105, i.e. less than 2-fold higher, was as effective as a dose of 60 MBq of 67Cu-DOTA-AE105 indicating that in clear contrast to the delivered doses 64Cu-DOTA-AE105 was surprisingly effective in treating human xenograft tumors (see example 4 and 5). This is ground-breaking as this allows for an approximately 2½ - 3-fold reduction in delivered dose in Gy when using 64Cu-DOTA-AE105 vs. 67Gy-DOTA-AE105. In other words, the calculated dose in the human dosimetry studies, where the maximal dose with respect to organs of 112 GBq leads to a radiation dose of 20.8 Gy which was deemed ineffective according to standard limits for target tumors doses of >30 Gy (palliative) or >50 Gy (curative), suddenly becomes relevant if the efficacy is 2½-3 fold more effective than the similar dose in Gy delivered by 67Cu-DOTA-AE105, i.e. the efficacy to be expected in humans of 20.8 Gy would be equivalent to >50 Gy (52-62) Gy, which would be highly relevant for clinical use.

### uPAR binding moieties

The uPAR binding moiety in the context of the present invention may be any entity providing targeting of 64Cu to uPAR within the human body, such as but not limited to antibodies, peptides, and small molecules towards uPAR whereby off target exposure to radiation is limited as much as possible.

One example of suitable uPAR binding moieties according to the present invention is antibodies (polyclonal antibodies, monoclonal antibodies) and antibody derived targeting moieties such as, scFv, Fab, nanobody, bispecific antibody, bifunctional antibody, diabody and minibody preserving their functional potential while being more limited in size.

In the context of large uPAR binding moiety such as antibodies, the targeting moiety provides the possibility for attaching more than one chelating agent thereby increasing the radionuclide load per conjugate. Thus, 64Cu labelled uPAR binding conjugates according to all aspects of the invention comprising one or more chelating agents, preferably two or more chelating agents when the targeting moiety is an antibody.

In one embodiment of all aspects of the invention, the uPAR binding moiety is selected from the group comprising antibodies (polyclonal antibodies or monoclonal antibodies), antibody derived targeting moieties such as, scFv, Fab, nanobody, bispecific antibody, bifunctional antibody, diabody and minibody.

Another example of suitable uPAR binding moieties according to the present invention is antibody mimetics, such as affibody molecules.

In one embodiment of all aspects of the invention, the uPAR binding moiety is an affibody molecule.

Another example of suitable uPAR binding moieties according to the present invention is nucleic acid molecules, such as aptamers.

In one embodiment of all aspects of the invention, the uPAR binding moiety is nucleic acid molecules, preferably aptamers.

Another example of suitable uPAR binding moieties according to the present invention is small molecules.

In one embodiment of all aspects of the invention, the targeting moiety is a small molecule.

Another example of suitable uPAR binding moieties is peptides or peptidomimetics which has shown promising potential for site specific targeting in of radionuclide-based therapy.

In a preferred embodiment of all aspects of the invention, the uPAR binding moiety is a peptide or peptidomimetics.

Another example of suitable uPAR binding moieties is amino-terminal fragments (ATF) of the natural ligand for uPAR, uPA, retaining the receptor binding domain of uPAR. In a preferred embodiment of all aspects of the invention, the uPAR binding moiety is amino-terminal fragments (ATF) of the natural ligand for uPAR, uPA, retaining the receptor binding domain of uPAR.

In context of the present invention the most preferred uPAR binding moieties are uPAR binding peptides.

### uPAR binding peptides

The uPAR binding peptides of the present invention may be in the form of a monomeric peptide, i.e., a single peptide or a multimeric peptide construct such as a dimer, trimer or tetramer. A multimeric peptide construct may be homomeric (i.e., consisting of identical peptides) or heteromeric (i.e., consisting of non-identical uPAR binding peptides) and multimeric peptide construct can possess higher affinity for a target receptor than its monomeric equivalent.

The uPAR binding peptides of the present invention may be synthesized using standard methods. One example of a standard method is described in US 7,026,282 as follows: the chain elongation steps of the Solid-phase peptide synthesis were carried out manually using polyethylene Syringes as reaction vessels. Synthesis was performed on KA-resin with preloaded Fmoc-amino acids on the acid labile linker. Five equivalents of Fmoc-amino acids activated by 1-hydroxy-7-benzotriazole (HOBt) and N,N'-diiso-propylcarbodiimide were used in the coupling steps and were allowed to react for more than 2 h. The Fmoc protecting group was removed with 20% piperidine in dimethyl formamide for 15-20 min. For cleavage, peptide-resins were treated for 1.5 h with 85% TFA containing 5% of phenol, mercaptoethanol, and thioanisole, respectively. The filtrates were concentrated by nitrogen flushing, and peptides were subsequently precipitated from, and washed four times with, diethyl ether. Peptides were finally dissolved/suspended in glacial acetic acid, lyophilised, redissolved in 10% acetic acid, and lyophilised again. Analytical HPLC analysis was performed on a Cs column using Waters 600E equipped with Waters photodiode array detector. A 25 min linear gradient from buffer A (0.1%TFA, 9.9% HO, 90% CHCN), was used. If considered necessary, peptides were purified on a preparative scale. The correct identities of the peptides were confirmed by matrix assisted laser desorption ionisation mass spectroscopy or electrospray ionization mass spectrometry. The purity was checked by reverse phase HPLC.

The "multimeric' peptides may be synthesised by standardised peptide synthesis methodologies using an orthogonal protection strategy (e.g., as described by Cwirla et al. Science, Vol 276, 1997, pp 1696-1699). Alternatively, heterogeneous methods well known to peptide chemists for construction of e.g. multimeric antigens for immunisation (D. N. Posnett et al (1988) J. Biol. Chem. 263:1719-1725) are also applicable within the present invention.

In one embodiment of all aspects of the invention, the uPAR binding peptides of the present invention comprises the sequence **([beta]-cyclohexyl-L-alanine)-(Phe)**-X-X-X, where the residues in bold are hot spot for interaction with uPAR, x designate any amino acid. Thereby providing binding of the peptide to its target uPAR. Preferably, such peptide are linear peptides having a length 5 to 25 amino acids, preferably 7 to 15 amino acids, more preferred 8 to 12 amino acids, most preferred 9 amino acids.

More preferably, the uPAR binding peptides of all aspects of the present invention comprises ([beta]-cyclohexyl-L-alanine)-(Phe)-X¹-X²-X³-X⁴-X⁵, wherein
X¹ represents Ser or D-Ser;
X² represents D-Arg, Arg, Gln, D-Tyr, or Tyr;
X³ represents Tyr, Leu, fbetal-cyclohexyl-L-alanine, or N-(2,3-dimethoxybenzyl)glycine;
X⁴ represents Leu or D-Phe; and
X⁵ represents Trp, fbetal-2-naphthyl-L-alanine, N-(3-indolylethyl)glycine, N-benzylglycine, N-(methylnaphthalyl)glycine, or N-(2,3-dimethoxybenzyl)glycine. Preferably, such peptide are linear peptides having a length 7 to 25 amino acids, preferably 7 to 15 amino acids, more preferred 8 to 12 amino acids, most preferred 9 amino acids.

Yet more preferably, the uPAR binding peptides comprises the binding hotspot of x-**Cha-Phe**-x-x-x-**Leu**-**Trp**-x, where the residues in bold are hot spots for interaction with uPAR, Cha is Cyclohexyl-(L)alanine, preferably [beta]-cyclohexyl-L-alanine, and x designate any amino acid. Thereby providing stable binding of the peptide to its target uPAR. Preferably, such peptide are linear peptides having a length 9 to 25 amino acids, preferably 9 to 15 amino acids, more preferred 9 to 12 amino acids, most preferred 9 amino acids.

These peptides in themselves, have high specificity towards the target uPAR as well as good affinity thereby facilitating sufficient retention of the resulting radionuclide labelled uPAR binding peptide conjugates at the target for the radionuclide to have sufficient time to provide a therapeutic relevant radioactive exposure of the targeted area. Furthermore, the peptides provide good compatibility with suitable chelating agents including DOTA, DOTAM, NOTA, NODAGA, SARCOPHAGINE, CB-TE2A and derivatives thereof, in particular DOTA.

In a preferred embodiment of all aspects off the present invention, the uPAR binding peptide is a monomeric peptide or multimeric peptide wherein the peptide constituent(s) is/are selected from the group consisting of:
(D-Asp)-([beta]-cyclohexyl-L-alanine)-(Phe)-(D-Ser)-(D-Arg)-(Tyr)-(Leu)-(Trp)-(Ser),
(Ser)-(Leu)-([beta]-cyclohexyl-L-alanine)-(Phe)-(D-Ser)-(Gln)-(Tyr)(Leu)-(Trp)-(Ser),
(D-Glu)-([beta]-cyclohexyl-L-alanine)-(Phe)-(D-Ser)-(D-Tyr)-(Tyr)-(Leu)-(Trp)-(Ser),
(Asp)-([beta]-cyclohexyl-L-alanine)-(Phe)-(D-Ser)-(D-Arg)-(Tyr)-(Leu)-(Trp)-(Ser),
(Asp)-([beta]-cyclohexyl-L-alanine)-(Phe)-(Ser)-(D-Arg)-(Tyr)-Leu)-(Trp)-(Ser),
(D-Asp)-([beta]-cyclohexyl-L-alanine)-(Phe)-(Ser)-(D-Arg)-(Tyr)-Leu)-(Trp)-(Ser),
(D-Thr)-([beta]-cyclohexyl-L-alanine)-(Phe)-(D-Ser)-(D-Arg)-(Tyr)-(Leu)-(Trp)-(Ser),
(D-Asp)-([beta]-cyclohexyl-L-alanine)-(Phe)-(D-Ser)-(D-Arg)-(Tyr)-(Leu)-([beta]-2-naphthyl-L-alanine)-(Ser),
(Asp)-([beta]-cyclohexyl-L-alanine)-(Phe)-(D-Ser)-(Arg)-(Tyr)-(Leu)-(Trp)-(Ser),
(Asp)-([beta]-cyclohexyl-L-alanine)-(Phe)-(D-Ser)-(D-Arg)-(Tyr)-(Leu)([beta]-1-naphthyl-L-alanine)-(Ser),
(D-Glu)-([beta]-cyclohexyl-L-alanine)-(Phe)-(D-Ser)-(Tyr)-(Tyr)-(Leu)-(Trp)-(Ser),
(Asp)-([beta]-cyclohexyl-L-alanine)-(Phe)-(D-Ser)-(D-Arg)-(Leu)-(Leu)-(Trp)-(D-His),
(Asp)-([beta]-cyclohexyl-L-alanine)-(Phe)-(D-Ser)-(D-Arg)-([beta]-cyclohexyl-L-alanine)-(Leu)-(Trp)-(Ile),
(Asp)-([beta]-cyclohexyl-L-alanine)-(Phe)-(D-Ser)-(D-Arg)-(Tyr)-(Leu)([beta]-1-naphthyl-L-alanine)-(D-His),
(Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine),and
(Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(lle).

In a preferred embodiment of all aspects of the invention, the uPAR binding peptide is a monomeric peptide selected from the group consisting of:
(D-Asp)-([beta]-cyclohexyl-L-alanine)-(Phe)-(D-Ser)-(D-Arg)-(Tyr)-(Leu)-(Trp)-(Ser),
(Ser)-(Leu)-([beta]-cyclohexyl-L-alanine)-(Phe)-(D-Ser)-(Gln)-(Tyr)(Leu)-(Trp)-(Ser),
(D-Glu)-([beta]-cyclohexyl-L-alanine)-(Phe)-(D-Ser)-(D-Tyr)-(Tyr)-(Leu)-(Trp)-(Ser),
(Asp)-([beta]-cyclohexyl-L-alanine)-(Phe)-(D-Ser)-(D-Arg)-(Tyr)-(Leu)-(Trp)-(Ser),
(Asp)-([beta]-cyclohexyl-L-alanine)-(Phe)-(Ser)-(D-Arg)-(Tyr)-Leu)-(Trp)-(Ser),
(D-Asp)-([beta]-cyclohexyl-L-alanine)-(Phe)-(Ser)-(D-Arg)-(Tyr)-Leu)-(Trp)-(Ser),
(D-Thr)-([beta]-cyclohexyl-L-alanine)-(Phe)-(D-Ser)-(D-Arg)-(Tyr)-(Leu)-(Trp)-(Ser),
(D-Asp)-([beta]-cyclohexyl-L-alanine)-(Phe)-(D-Ser)-(D-Arg)-(Tyr)-(Leu)-([beta]-2-naphthyl-L-alanine)-(Ser),
(Asp)-([beta]-cyclohexyl-L-alanine)-(Phe)-(D-Ser)-(Arg)-(Tyr)-(Leu)-(Trp)-(Ser),
(Asp)-([beta]-cyclohexyl-L-alanine)-(Phe)-(D-Ser)-(D-Arg)-(Tyr)-(Leu)([beta]-1-naphthyl-L-alanine)-(Ser),
(D-Glu)-([beta]-cyclohexyl-L-alanine)-(Phe)-(D-Ser)-(Tyr)-(Tyr)-(Leu)-(Trp)-(Ser),
(Asp)-([beta]-cyclohexyl-L-alanine)-(Phe)-(D-Ser)-(D-Arg)-(Leu)-(Leu)-(Trp)-(D-His),
(Asp)-([beta]-cyclohexyl-L-alanine)-(Phe)-(D-Ser)-(D-Arg)-([beta]-cyclohexyl-L-alanine)-(Leu)-(Trp)-(Ile),
(Asp)-([beta]-cyclohexyl-L-alanine)-(Phe)-(D-Ser)-(D-Arg)-(Tyr)-(Leu)([beta]-1-naphthyl-L-alanine)-(D-His),
(Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine),and
(Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(lle).

These peptides all share a minimum structure by having at least part of the binding hotspot of x-**Cha**-**Phe**-x-x-x-**Leu**-**Trp**-x, where the residues in bold are hot spots for interaction with uPAR, Cha is Cyclohexyl-(L)alanine, and x designate any amino acid. These peptides in themselves, have high specificity towards the target uPAR as well as good affinity thereby facilitating sufficient retention of the resulting radionuclide labelled uPAR binding peptide conjugates at the target for the radionuclide to have sufficient time to provide a therapeutic relevant radioactive exposure of the targeted area. Furthermore, the peptides provide good compatibility with suitable chelating agents including DOTA, DOTAM, NOTA, NODAGA, SARCOPHAGINE, CB-TE2A and derivatives thereof, in particular DOTA.

In a most preferred embodiment, the uPAR binding peptide is (D-Asp)-([beta]-cyclohexyl-L-alanine)-(Phe)-(D-Ser)-(D-Arg)-(Tyr)-(Leu)-(Trp)-(Ser) also designated AE105 in the context of the present invention. Thereby providing a uPAR binding peptide which in itself has superior specificity towards the target uPAR as well as high affinity thereby facilitating superior retention of the radionuclide labelled uPAR binding peptide conjugates at the target for the radionuclide to have sufficient time to provide a therapeutic relevant radioactive exposure of the targeted area. Furthermore, the uPAR binding peptide provides good compatibility with suitable chelating agents including DOTA, DOTAM, NOTA, NODAGA, SARCOPHAGINE, CB-TE2A and derivatives thereof, in particular DOTA and superior target binding capabilities when labelled with the radionuclide 64Cu.

Radiolabeling of the peptide(s) selected for the radionuclide labelled uPAR binding peptide conjugates of all aspects of the present invention is enabled by coupling a chelating agent suitable for binding radiometals to the peptide. The chelating agent is capable of binding a selected radionuclide thereto.

### Chelator

The use of various chelating agents suitable for binding radiometals for radio labeling of targeting moieties, such as peptides, is well known in the art. Suitable chelating agents generally include those which contain a tetradentate ligand with at least one sulfur group available for binding the metal radionuclide such as the known N3S and N2S2 ligands. The chelating agent is coupled to the uPAR binding moiety, such as a peptide, by standard methodology known in the field of the invention and may be added at any location on the uPAR binding moiety, provided that the biological activity such as binding properties and specificity of the uPAR binding moiety is not adversely affected. Preferably, the chelating group is, in the context of the uPAR binding moiety being a peptide, covalently coupled to the amino terminal amino acid of the peptide. The chelating group may advantageously be attached to the peptide during solid phase peptide synthesis or added by solution phase chemistry after the peptide has been obtained. Preferred chelating agents include DOTA (1,4,7, 10-tetrakis(carboxymethyl)-1,4,7,10 tetraazacyclo dodecane), DOTAM (2-[4,7,10-tris(2-amino-2-oxoethyl)-1,4,7,10-tetrazacyclododec-1-yl]acetamide), NOTA (2,2',2"-(1,4,7-triazacyclononane-1,4,7-triyl)triacetic acid), NODAGA, SARCOPHAGINE (3,6,10,13,16,19-hexaazabicyclo(6,6,6)icosane), CB-TE2A (1,4,8,11-Tetraazabicyclo[6.6.2]hexadecane-4,11-diacetic acid) and derivatives thereof, which constitute an important class of chelators for biomedical applications as they accommodate very stably a variety of di- and trivalent metal ions.

DOTA (Dodecane tetraacetic acid) with the chemical formula (1,4,7, 1O-tetrakis(carboxymethyl)-1,4,7,10 tetraazacyclo dodecane) and its derivatives, are particularly acknowledged to constitute an important class of chelators for biomedical applications as they accommodate very stably a variety of di- and trivalent metal ions, including 64Cu.

DOTAM also known as TCMC is a DOTA analogue.

SARCOPHAGINE (Sar) is a bicyclic cage-like chelator molecule derived from cyclam. The chemical formula of sarcophagine is 3,6,10,13,16,19-hexaazabicyclo(6,6,6)icosane and additional functional or non-functional groups are often linked to this structure, thereby creating derivatives of sarcophagine, such as in DiAmSar (1,8-diamino-Sar), AmBaSar (4-((8-amino-3,6,10,13,16,19-hexaazabicyclo [6.6.6] icosane-1-ylamino)methyl)benzoic acid), and MeCOSar (5-(8-methyl-3,6,10,13,16,19-hexaaza-bicyclo[6.6.6]icosan-1-ylamino)-5-oxopentanoic acid). Sarcophagine and derivatives thereof are suitable chelating agents for use in the radionuclide labelled uPAR binding conjugates of all aspects of the present invention and are preferred for 64Cu labelled uPAR binding conjugates.

In one embodiment of all aspects of the present invention, the chelating agent is selected from the group consisting of DOTA, DOTAM, NOTA, NODAGA, SARCOPHAGINE, CB-TE2A and derivatives thereof, preferably DOTA, DOTAM, NOTA, NODAGA, SARCOPHAGINE, DiAmSar, AmBaSar, MeCOSar, or CB-TE2A.

In a preferred embodiment, the chelating agent is selected from the group consisting of DOTA, DOTAM, SARCOPHAGINE, and derivatives thereof, preferably DOTA, DOTAM, SARCOPHAGINE, DiAmSar, AmBaSar, or MeCOSar.

In a more preferred embodiment, the chelating agent is DOTA.

In one embodiment of all aspects of the present invention, the chelating agent of the uPAR binding conjugate is DOTA, and the radionuclide is 64Cu.

In one embodiment of all aspects of the present invention, the chelating agent is DOTA and the uPAR binding peptide is selected from the group consisting of (D-Asp)-([beta]-cyclohexyl-L-alanine)-(Phe)-(D-Ser)-(D-Arg)-(Tyr)-(Leu)-(Trp)-(Ser),
(Ser)-(Leu)-([beta]-cyclohexyl-L-alanine)-(Phe)-(D-Ser)-(Gln)-(Tyr)(Leu)-(Trp)-(Ser),
(D-Glu)-([beta]-cyclohexyl-L-alanine)-(Phe)-(D-Ser)-(D-Tyr)-(Tyr)-(Leu)-(Trp)-(Ser),
(Asp)-([beta]-cyclohexyl-L-alanine)-(Phe)-(D-Ser)-(D-Arg)-(Tyr)-(Leu)-(Trp)-(Ser),
(Asp)-([beta]-cyclohexyl-L-alanine)-(Phe)-(Ser)-(D-Arg)-(Tyr)-Leu)-(Trp)-(Ser),
(D-Asp)-([beta]-cyclohexyl-L-alanine)-(Phe)-(Ser)-(D-Arg)-(Tyr)-Leu)-(Trp)-(Ser),
(D-Thr)-([beta]-cyclohexyl-L-alanine)-(Phe)-(D-Ser)-(D-Arg)-(Tyr)-(Leu)-(Trp)-(Ser),
(D-Asp)-([beta]-cyclohexyl-L-alanine)-(Phe)-(D-Ser)-(D-Arg)-(Tyr)-(Leu)-([beta]-2-naphthyl-L-alanine)-(Ser),
(Asp)-([beta]-cyclohexyl-L-alanine)-(Phe)-(D-Ser)-(Arg)-(Tyr)-(Leu)-(Trp)-(Ser),
(Asp)-([beta]-cyclohexyl-L-alanine)-(Phe)-(D-Ser)-(D-Arg)-(Tyr)-(Leu)([beta]-1-naphthyl-L-alanine)-(Ser),
(D-Glu)-([beta]-cyclohexyl-L-alanine)-(Phe)-(D-Ser)-(Tyr)-(Tyr)-(Leu)-(Trp)-(Ser),
(Asp)-([beta]-cyclohexyl-L-alanine)-(Phe)-(D-Ser)-(D-Arg)-(Leu)-(Leu)-(Trp)-(D-His),
(Asp)-([beta]-cyclohexyl-L-alanine)-(Phe)-(D-Ser)-(D-Arg)-([beta]-cyclohexyl-L-alanine)-(Leu)-(Trp)-(Ile),
(Asp)-([beta]-cyclohexyl-L-alanine)-(Phe)-(D-Ser)-(D-Arg)-(Tyr)-(Leu)([beta]-1-naphthyl-L-alanine)-(D-His),
(Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine),and
(Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(lle), and the radionuclide is 64Cu.

In a preferred embodiment, the chelating agent is DOTA and the uPAR binding peptide is (D-Asp)-([beta]-cyclohexyl-L-alanine)-(Phe)-(D-Ser)-(D-Arg)-(Tyr)-(Leu)-(Trp)-(Ser) and the radionuclide is 64Cu.

In one embodiment of all aspects of the present invention, the chelating agent of the uPAR binding conjugate is DOTAM and the radionuclide is 64Cu.

In one embodiment of all aspects of the present invention, the chelating agent is DOTAM and the uPAR binding peptide is selected from the group consisting of (D-Asp)-([beta]-cyclohexyl-L-alanine)-(Phe)-(D-Ser)-(D-Arg)-(Tyr)-(Leu)-(Trp)-(Ser),
(Ser)-(Leu)-([beta]-cyclohexyl-L-alanine)-(Phe)-(D-Ser)-(Gln)-(Tyr)(Leu)-(Trp)-(Ser),
(D-Glu)-([beta]-cyclohexyl-L-alanine)-(Phe)-(D-Ser)-(D-Tyr)-(Tyr)-(Leu)-(Trp)-(Ser),
(Asp)-([beta]-cyclohexyl-L-alanine)-(Phe)-(D-Ser)-(D-Arg)-(Tyr)-(Leu)-(Trp)-(Ser),
(Asp)-([beta]-cyclohexyl-L-alanine)-(Phe)-(Ser)-(D-Arg)-(Tyr)-Leu)-(Trp)-(Ser),
(D-Asp)-([beta]-cyclohexyl-L-alanine)-(Phe)-(Ser)-(D-Arg)-(Tyr)-Leu)-(Trp)-(Ser),
(D-Thr)-([beta]-cyclohexyl-L-alanine)-(Phe)-(D-Ser)-(D-Arg)-(Tyr)-(Leu)-(Trp)-(Ser),
(D-Asp)-([beta]-cyclohexyl-L-alanine)-(Phe)-(D-Ser)-(D-Arg)-(Tyr)-(Leu)-([beta]-2-naphthyl-L-alanine)-(Ser),
(Asp)-([beta]-cyclohexyl-L-alanine)-(Phe)-(D-Ser)-(Arg)-(Tyr)-(Leu)-(Trp)-(Ser),
(Asp)-([beta]-cyclohexyl-L-alanine)-(Phe)-(D-Ser)-(D-Arg)-(Tyr)-(Leu)([beta]-1-naphthyl-L-alanine)-(Ser),
(D-Glu)-([beta]-cyclohexyl-L-alanine)-(Phe)-(D-Ser)-(Tyr)-(Tyr)-(Leu)-(Trp)-(Ser),
(Asp)-([beta]-cyclohexyl-L-alanine)-(Phe)-(D-Ser)-(D-Arg)-(Leu)-(Leu)-(Trp)-(D-His),
(Asp)-([beta]-cyclohexyl-L-alanine)-(Phe)-(D-Ser)-(D-Arg)-([beta]-cyclohexyl-L-alanine)-(Leu)-(Trp)-(Ile),
(Asp)-([beta]-cyclohexyl-L-alanine)-(Phe)-(D-Ser)-(D-Arg)-(Tyr)-(Leu)([beta]-1-naphthyl-L-alanine)-(D-His),
(Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine),and
(Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(lle),
And the radionuclide is 64Cu.

In a preferred embodiment, the chelating agent is DOTAM and the uPAR binding peptide is (D-Asp)-([beta]-cyclohexyl-L-alanine)-(Phe)-(D-Ser)-(D-Arg)-(Tyr)-(Leu)-(Trp)-(Ser) and the radionuclide is 64Cu.

In one embodiment of all aspects of the present invention, the chelating agent of the uPAR binding conjugate is NOTA, and the radionuclide is 64Cu.

In one embodiment of all aspects of the present invention, the chelating agent is NOTA and the uPAR binding peptide is selected from the group consisting of (D-Asp)-([beta]-cyclohexyl-L-alanine)-(Phe)-(D-Ser)-(D-Arg)-(Tyr)-(Leu)-(Trp)-(Ser),
(Ser)-(Leu)-([beta]-cyclohexyl-L-alanine)-(Phe)-(D-Ser)-(Gln)-(Tyr)(Leu)-(Trp)-(Ser),
(D-Glu)-([beta]-cyclohexyl-L-alanine)-(Phe)-(D-Ser)-(D-Tyr)-(Tyr)-(Leu)-(Trp)-(Ser),
(Asp)-([beta]-cyclohexyl-L-alanine)-(Phe)-(D-Ser)-(D-Arg)-(Tyr)-(Leu)-(Trp)-(Ser),
(Asp)-([beta]-cyclohexyl-L-alanine)-(Phe)-(Ser)-(D-Arg)-(Tyr)-Leu)-(Trp)-(Ser),
(D-Asp)-([beta]-cyclohexyl-L-alanine)-(Phe)-(Ser)-(D-Arg)-(Tyr)-Leu)-(Trp)-(Ser),
(D-Thr)-([beta]-cyclohexyl-L-alanine)-(Phe)-(D-Ser)-(D-Arg)-(Tyr)-(Leu)-(Trp)-(Ser),
(D-Asp)-([beta]-cyclohexyl-L-alanine)-(Phe)-(D-Ser)-(D-Arg)-(Tyr)-(Leu)-([beta]-2-naphthyl-L-alanine)-(Ser),
(Asp)-([beta]-cyclohexyl-L-alanine)-(Phe)-(D-Ser)-(Arg)-(Tyr)-(Leu)-(Trp)-(Ser),
(Asp)-([beta]-cyclohexyl-L-alanine)-(Phe)-(D-Ser)-(D-Arg)-(Tyr)-(Leu)([beta]-1-naphthyl-L-alanine)-(Ser),
(D-Glu)-([beta]-cyclohexyl-L-alanine)-(Phe)-(D-Ser)-(Tyr)-(Tyr)-(Leu)-(Trp)-(Ser),
(Asp)-([beta]-cyclohexyl-L-alanine)-(Phe)-(D-Ser)-(D-Arg)-(Leu)-(Leu)-(Trp)-(D-His),
(Asp)-([beta]-cyclohexyl-L-alanine)-(Phe)-(D-Ser)-(D-Arg)-([beta]-cyclohexyl-L-alanine)-(Leu)-(Trp)-(Ile),
(Asp)-([beta]-cyclohexyl-L-alanine)-(Phe)-(D-Ser)-(D-Arg)-(Tyr)-(Leu)([beta]-1-naphthyl-L-alanine)-(D-His),
(Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine),and
(Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(lle),
And the radionuclide is 64Cu.

In a preferred embodiment, the chelating agent is NOTA and the uPAR binding peptide is (D-Asp)-([beta]-cyclohexyl-L-alanine)-(Phe)-(D-Ser)-(D-Arg)-(Tyr)-(Leu)-(Trp)-(Ser), and the radionuclide is 64Cu.

In one embodiment of all aspects of the present invention, the chelating agent of the uPAR binding conjugate is NODAGA and the radionuclide is 64Cu.

In one embodiment of all aspects of the present invention, the chelating agent is NODAGA and the uPAR binding peptide is selected from the group consisting of (D-Asp)-([beta]-cyclohexyl-L-alanine)-(Phe)-(D-Ser)-(D-Arg)-(Tyr)-(Leu)-(Trp)-(Ser),
(Ser)-(Leu)-([beta]-cyclohexyl-L-alanine)-(Phe)-(D-Ser)-(Gln)-(Tyr)(Leu)-(Trp)-(Ser),
(D-Glu)-([beta]-cyclohexyl-L-alanine)-(Phe)-(D-Ser)-(D-Tyr)-(Tyr)-(Leu)-(Trp)-(Ser),
(Asp)-([beta]-cyclohexyl-L-alanine)-(Phe)-(D-Ser)-(D-Arg)-(Tyr)-(Leu)-(Trp)-(Ser),
(Asp)-([beta]-cyclohexyl-L-alanine)-(Phe)-(Ser)-(D-Arg)-(Tyr)-Leu)-(Trp)-(Ser),
(D-Asp)-([beta]-cyclohexyl-L-alanine)-(Phe)-(Ser)-(D-Arg)-(Tyr)-Leu)-(Trp)-(Ser),
(D-Thr)-([beta]-cyclohexyl-L-alanine)-(Phe)-(D-Ser)-(D-Arg)-(Tyr)-(Leu)-(Trp)-(Ser),
(D-Asp)-([beta]-cyclohexyl-L-alanine)-(Phe)-(D-Ser)-(D-Arg)-(Tyr)-(Leu)-([beta]-2-naphthyl-L-alanine)-(Ser),
(Asp)-([beta]-cyclohexyl-L-alanine)-(Phe)-(D-Ser)-(Arg)-(Tyr)-(Leu)-(Trp)-(Ser),
(Asp)-([beta]-cyclohexyl-L-alanine)-(Phe)-(D-Ser)-(D-Arg)-(Tyr)-(Leu)([beta]-1-naphthyl-L-alanine)-(Ser),
(D-Glu)-([beta]-cyclohexyl-L-alanine)-(Phe)-(D-Ser)-(Tyr)-(Tyr)-(Leu)-(Trp)-(Ser),
(Asp)-([beta]-cyclohexyl-L-alanine)-(Phe)-(D-Ser)-(D-Arg)-(Leu)-(Leu)-(Trp)-(D-His),
(Asp)-([beta]-cyclohexyl-L-alanine)-(Phe)-(D-Ser)-(D-Arg)-([beta]-cyclohexyl-L-alanine)-(Leu)-(Trp)-(Ile),
(Asp)-([beta]-cyclohexyl-L-alanine)-(Phe)-(D-Ser)-(D-Arg)-(Tyr)-(Leu)([beta]-1-naphthyl-L-alanine)-(D-His),
(Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine),and
(Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(lle),
and the radionuclide is 64Cu.

In a preferred embodiment, the chelating agent is NODAGA and the uPAR binding peptide is (D-Asp)-([beta]-cyclohexyl-L-alanine)-(Phe)-(D-Ser)-(D-Arg)-(Tyr)-(Leu)-(Trp)-(Ser), and the radionuclide is 64Cu.

In one embodiment of all aspects of the present invention, the chelating agent of the uPAR binding conjugate is MeCOSar and the radionuclide is 64Cu.

In one embodiment of all aspects of the present invention, the chelating agent is MeCOSar and the uPAR binding peptide is selected from the group consisting of (D-Asp)-([beta]-cyclohexyl-L-alanine)-(Phe)-(D-Ser)-(D-Arg)-(Tyr)-(Leu)-(Trp)-(Ser),
(Ser)-(Leu)-([beta]-cyclohexyl-L-alanine)-(Phe)-(D-Ser)-(Gln)-(Tyr)(Leu)-(Trp)-(Ser),
(D-Glu)-([beta]-cyclohexyl-L-alanine)-(Phe)-(D-Ser)-(D-Tyr)-(Tyr)-(Leu)-(Trp)-(Ser),
(Asp)-([beta]-cyclohexyl-L-alanine)-(Phe)-(D-Ser)-(D-Arg)-(Tyr)-(Leu)-(Trp)-(Ser),
(Asp)-([beta]-cyclohexyl-L-alanine)-(Phe)-(Ser)-(D-Arg)-(Tyr)-Leu)-(Trp)-(Ser),
(D-Asp)-([beta]-cyclohexyl-L-alanine)-(Phe)-(Ser)-(D-Arg)-(Tyr)-Leu)-(Trp)-(Ser),
(D-Thr)-([beta]-cyclohexyl-L-alanine)-(Phe)-(D-Ser)-(D-Arg)-(Tyr)-(Leu)-(Trp)-(Ser),
(D-Asp)-([beta]-cyclohexyl-L-alanine)-(Phe)-(D-Ser)-(D-Arg)-(Tyr)-(Leu)-([beta]-2-naphthyl-L-alanine)-(Ser),
(Asp)-([beta]-cyclohexyl-L-alanine)-(Phe)-(D-Ser)-(Arg)-(Tyr)-(Leu)-(Trp)-(Ser),
(Asp)-([beta]-cyclohexyl-L-alanine)-(Phe)-(D-Ser)-(D-Arg)-(Tyr)-(Leu)([beta]-1-naphthyl-L-alanine)-(Ser),
(D-Glu)-([beta]-cyclohexyl-L-alanine)-(Phe)-(D-Ser)-(Tyr)-(Tyr)-(Leu)-(Trp)-(Ser),
(Asp)-([beta]-cyclohexyl-L-alanine)-(Phe)-(D-Ser)-(D-Arg)-(Leu)-(Leu)-(Trp)-(D-His),
(Asp)-([beta]-cyclohexyl-L-alanine)-(Phe)-(D-Ser)-(D-Arg)-([beta]-cyclohexyl-L-alanine)-(Leu)-(Trp)-(Ile),
(Asp)-([beta]-cyclohexyl-L-alanine)-(Phe)-(D-Ser)-(D-Arg)-(Tyr)-(Leu)([beta]-1-naphthyl-L-alanine)-(D-His),
(Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine),and
(Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(lle),
and the radionuclide is 64Cu.

In a preferred embodiment, the chelating agent is MeCOSar and the uPAR binding peptide is (D-Asp)-([beta]-cyclohexyl-L-alanine)-(Phe)-(D-Ser)-(D-Arg)-(Tyr)-(Leu)-(Trp)-(Ser), and the radionuclide is 64Cu.

In one embodiment of all aspects of the present invention, the chelating agent of the uPAR binding conjugate is CB-TE2A, and the radionuclide is 64Cu.

In one embodiment of all aspects of the present invention, the chelating agent is CB-TE2A and the uPAR binding peptide is selected from the group consisting of (D-Asp)-([beta]-cyclohexyl-L-alanine)-(Phe)-(D-Ser)-(D-Arg)-(Tyr)-(Leu)-(Trp)-(Ser),
(Ser)-(Leu)-([beta]-cyclohexyl-L-alanine)-(Phe)-(D-Ser)-(Gln)-(Tyr)(Leu)-(Trp)-(Ser),
(D-Glu)-([beta]-cyclohexyl-L-alanine)-(Phe)-(D-Ser)-(D-Tyr)-(Tyr)-(Leu)-(Trp)-(Ser),
(Asp)-([beta]-cyclohexyl-L-alanine)-(Phe)-(D-Ser)-(D-Arg)-(Tyr)-(Leu)-(Trp)-(Ser),
(Asp)-([beta]-cyclohexyl-L-alanine)-(Phe)-(Ser)-(D-Arg)-(Tyr)-Leu)-(Trp)-(Ser),
(D-Asp)-([beta]-cyclohexyl-L-alanine)-(Phe)-(Ser)-(D-Arg)-(Tyr)-Leu)-(Trp)-(Ser),
(D-Thr)-([beta]-cyclohexyl-L-alanine)-(Phe)-(D-Ser)-(D-Arg)-(Tyr)-(Leu)-(Trp)-(Ser),
(D-Asp)-([beta]-cyclohexyl-L-alanine)-(Phe)-(D-Ser)-(D-Arg)-(Tyr)-(Leu)-([beta]-2-naphthyl-L-alanine)-(Ser),
(Asp)-([beta]-cyclohexyl-L-alanine)-(Phe)-(D-Ser)-(Arg)-(Tyr)-(Leu)-(Trp)-(Ser),
(Asp)-([beta]-cyclohexyl-L-alanine)-(Phe)-(D-Ser)-(D-Arg)-(Tyr)-(Leu)([beta]-1-naphthyl-L-alanine)-(Ser),
(D-Glu)-([beta]-cyclohexyl-L-alanine)-(Phe)-(D-Ser)-(Tyr)-(Tyr)-(Leu)-(Trp)-(Ser),
(Asp)-([beta]-cyclohexyl-L-alanine)-(Phe)-(D-Ser)-(D-Arg)-(Leu)-(Leu)-(Trp)-(D-His),
(Asp)-([beta]-cyclohexyl-L-alanine)-(Phe)-(D-Ser)-(D-Arg)-([beta]-cyclohexyl-L-alanine)-(Leu)-(Trp)-(Ile),
(Asp)-([beta]-cyclohexyl-L-alanine)-(Phe)-(D-Ser)-(D-Arg)-(Tyr)-(Leu)([beta]-1-naphthyl-L-alanine)-(D-His),
(Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine),and
(Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(lle),
and the radionuclide is 64Cu.

In a preferred embodiment, the chelating agent is CB-TE2A and the uPAR binding peptide is (D-Asp)-([beta]-cyclohexyl-L-alanine)-(Phe)-(D-Ser)-(D-Arg)-(Tyr)-(Leu)-(Trp)-(Ser), and the radionuclide is 64Cu.

### Diseases

The inventor has shown therapeutic efficacy of a 64Cu labelled uPAR binding conjugate in two different uPAR expressing cancer diseases. It is generally accepted that therapeutic efficacy across two or more different cancer diseases constitutes a very strong support for therapeutic efficacy in general for cancers expressing the relevant target. Thus, the 64Cu labelled uPAR binding conjugate of the present invention allows for treatment of uPAR expressing cancers, in particular cancer types having high expression of uPAR such as breast cancer, Brain cancer, in particular high grade gliomas, glioblastoma, NSCLC (non-small cell lung cancer), SCLC (small cell lung cancer), Neuroendocrine tumors, Head and neck cancer, HNSCC (Head and Neck squamous cell carcinoma), pancreas cancer, prostate cancer, CRC (Colorectal cancer) and gastric cancer.

In one embodiment of all aspects of the present invention, the uPAR expressing cancer is selected from the group consisting of breast cancer, Brain cancer (in particular high grade gliomas, glioblastoma, and meningioma), NSCLC (non-small cell lung cancer), SCLC (small cell lung cancer), Neuroendocrine tumors, Head and neck cancer, HNSCC (Head and Neck squamous cell carcinoma), pancreas cancer, liver cancer, prostate cancer, CRC (Colorectal cancer), urinary bladder cancer, ovarian cancer, cervical cancer, melanoma, esophageal cancer and gastric cancer, preferably breast cancer, NSCLC, Neuroendocrine tumors, HNSCC, pancreas cancer, prostate cancer, CRC, glioblastoma, urinary bladder cancer and gastric cancer, more preferably NSCLC, CRC, urinary bladder cancer and glioblastoma, more preferred urinary bladder cancer and glioblastoma and most preferred glioblastoma.

In a preferred embodiment of all aspects of the present invention, the uPAR expressing cancer is selected from the group consisting of high-grade glioma and urinary bladder cancer.

In a most preferred embodiment of all aspects of the present invention, the uPAR expressing cancer is glioblastoma.

### Dosage and route of administration

Radionuclide labelled uPAR binding conjugates according to the present invention may be administered systemically to a patient, preferably a human patient. Systemic administration of radionuclide labelled uPAR binding conjugates according to all aspects of the present invention are typically administered intravenously and may be administered intravenously in any conventional medium for intravenous injection.

Suitable cumulative doses for treatment of uPAR expressing cancer in human patients are in the range of 40 GBq to 200 GBq, such as 40 GBq to 163 GBq, preferably 50 GBq to 112 GBq whereby a good therapeutic effect is achieved while minimizing side effects.

In one embodiment of all aspects of the present invention, the cumulative dose administered intravenously to a human patient of the 64Cu labeled uPAR binding conjugate according to any of the previous embodiments is in the range of 40 GBq to 200 GBq, such as 40 GBq to 163 GBq, preferably 50 GBq to 112 GBq.

In some cancer diseases, systemic exposure to radionuclide-based therapy, e.g. by intravenous administration, does not provide optimal targeting of the cancer and localized administration may provide for better targeting of the cancer tissue. Localized administration may be provided by direct injection into the tumor, installation into a hollow organ, e.g. the urinary bladder, or by using a catheter or other suitable tubing providing access to the relevant site of administration or administration into the blood stream a site with delivery more directly to the tumor.

A specific example is administration of a dose of radionuclide labelled uPAR binding conjugate to the urinary bladder, by bladder installation, whereby a suitable volume of liquid comprising a single dose of approximately 2GBq to 10GBq of the radionuclide labelled uPAR binding conjugate is delivered to the bladder lumen and maintained there for a period of time such as 10 minutes to 60 minutes before draining the bladder. The administration is repeated a suitable number of times such as 5 to 35 doses in order to reach a desired cumulative dose. Suitable cumulative doses for localized treatment of the bladder are 335GBq installed for 1 h or 1,340 GBq installed for 15 min. suitable ranges of cumulative doses for localized treatment of the bladder are,
up to 335GBq installed for 15 min to 2 h, such as for 15min to 1 h or up to 1,340GBq if installed for up to 15 min, such as 5 min to 15 min,
preferably in the range of 50-335GBq installed for 15 min to 2 h, such as for 15min to 1 h or 200-1,340GBq if installed for up to 15 min, such as 5 min to 15 min,
most preferred in the range of 100-335GBq installed for 15 min to 2 h, such as for 15min to 1 h or 400-1,340GBq if installed for up to 15 min, such as 5 min to 15 min.

In one embodiment of all aspects of the present invention, the cumulative dose administered locally to the bladder of a human patient of the 64Cu labeled uPAR binding conjugate according to any of the previous embodiments is up to 335GBq installed for up to 1 h (or 1,340GBq if installed for up to 15 min, such as 10 min to 15 min. Preferably, single doses of 2 GBq to 20 GBq are administered to the bladder lumen and maintained there for 10 min to 60min before draining of the bladder, and the administration is repeated 5 to 35 times until the desired cumulative dose is achieved.

Another specific example is administration of a dose of radionuclide labelled uPAR binding conjugate locally to the brain. Local administration to the brain may be provided by use of suitable catheter systems where the placement of the catheter is obtained through stereotactic technique, and whereby a suitable volume of liquid comprising the radionuclide labelled uPAR binding conjugate is delivered locally to the brain. When combined with pressure infusion the technique is also known as convection-enhanced diffusion.

The local administration may also be achieved by intra-arterial administration to the brain, also known as super-selective intra-arterial cerebral infusion (SSIACI) where delivery is through a targeted vascular territory. In addition, these techniques may be combined with blood-brain barrier disruption such as mannitol or Focused Ultrasound.

### Embodiments

Embodiment 1, a radionuclide labelled uPAR binding conjugate for use in treatment of uPAR expressing cancer, wherein said radionuclide labelled uPAR conjugate comprises:
a) A uPAR binding moiety
b) A chelating agent suitable for binding radiometals
c) The radionuclide 64Cu

And wherein the peptide is coupled to 64Cu by the chelating agent.

Embodiment 2, the radionuclide labelled uPAR binding conjugate for use according to embodiment 1, wherein said uPAR binding moiety is a uPAR binding peptide

Embodiment 3, the radionuclide labelled uPAR binding conjugate for use according any one of embodiments 1 to 2, wherein said radionuclide labelled uPAR binding conjugate consists of
a) A uPAR binding moiety, preferably a uPAR binding peptide
b) A chelating agent suitable for binding radiometals
c) The radionuclide 64Cu
And wherein the peptide is coupled to 64Cu by the chelating agent.

Embodiment 4, the radionuclide labelled uPAR binding conjugate for use according to any one of embodiments 2 to 3, wherein the uPAR binding peptide comprises the sequence **([beta]-cyclohexyl-L-alanine)-(Phe)**-X-X-X, wherein x designate any amino acid.

Embodiment 5, the radionuclide labelled uPAR binding conjugate for use according to any one of embodiments 2 to 4, wherein the uPAR binding peptide comprises the sequence x-Cyclohexyl-(L)alanine -Phe-x-x-x-Leu-Trp-x, wherein x designates any amino acid.

Embodiment 6, the radionuclide labelled uPAR binding conjugate for use according to any one of embodiments 2 to 5, wherein the uPAR binding peptide is a monomeric peptide or a multimeric peptide wherein the one or more peptide constituent independently has a length of 5 to 25 amino acids, preferably 7 to 15 amino acids, more preferred 8 to 12 amino acids, most preferred 9 amino acids.

Embodiment 7, the radionuclide labelled uPAR binding conjugate for use according to embodiment 6, wherein the one or more peptide constituent is independently selected from the group consisting of:
(D-Asp)-([beta]-cyclohexyl-L-alanine)-(Phe)-(D-Ser)-(D-Arg)-(Tyr)-(Leu)-(Trp)-(Ser),
(Ser)-(Leu)-([beta]-cyclohexyl-L-alanine)-(Phe)-(D-Ser)-(Gln)-(Tyr)(Leu)-(Trp)-(Ser),
(D-Glu)-([beta]-cyclohexyl-L-alanine)-(Phe)-(D-Ser)-(D-Tyr)-(Tyr)-(Leu)-(Trp)-(Ser),
(Asp)-([beta]-cyclohexyl-L-alanine)-(Phe)-(D-Ser)-(D-Arg)-(Tyr)-(Leu)-(Trp)-(Ser),
(Asp)-([beta]-cyclohexyl-L-alanine)-(Phe)-(Ser)-(D-Arg)-(Tyr)-Leu)-(Trp)-(Ser),
(D-Asp)-([beta]-cyclohexyl-L-alanine)-(Phe)-(Ser)-(D-Arg)-(Tyr)-Leu)-(Trp)-(Ser),
(D-Thr)-([beta]-cyclohexyl-L-alanine)-(Phe)-(D-Ser)-(D-Arg)-(Tyr)-(Leu)-(Trp)-(Ser),
(D-Asp)-([beta]-cyclohexyl-L-alanine)-(Phe)-(D-Ser)-(D-Arg)-(Tyr)-(Leu)-([beta]-2-naphthyl-L-alanine)-(Ser),
(Asp)-([beta]-cyclohexyl-L-alanine)-(Phe)-(D-Ser)-(Arg)-(Tyr)-(Leu)-(Trp)-(Ser),
(Asp)-([beta]-cyclohexyl-L-alanine)-(Phe)-(D-Ser)-(D-Arg)-(Tyr)-(Leu)([beta]-1-naphthyl-L-alanine)-(Ser),
(D-Glu)-([beta]-cyclohexyl-L-alanine)-(Phe)-(D-Ser)-(Tyr)-(Tyr)-(Leu)-(Trp)-(Ser),
(Asp)-([beta]-cyclohexyl-L-alanine)-(Phe)-(D-Ser)-(D-Arg)-(Leu)-(Leu)-(Trp)-(D-His),
(Asp)-([beta]-cyclohexyl-L-alanine)-(Phe)-(D-Ser)-(D-Arg)-([beta]-cyclohexyl-L-alanine)-(Leu)-(Trp)-(Ile),
(Asp)-([beta]-cyclohexyl-L-alanine)-(Phe)-(D-Ser)-(D-Arg)-(Tyr)-(Leu)([beta]-1-naphthyl-L-alanine)-(D-His),
(Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine),and
(Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(lle).

Embodiment 8, the radionuclide labelled uPAR binding conjugate for use according to any one of embodiments 2 to 7, wherein the uPAR binding peptide is (D-Asp)-([beta]-cyclohexyl-L-alanine)-(Phe)-(D-Ser)-(D-Arg)-(Tyr)-(Leu)-(Trp)-(Ser)

Embodiment 9, the radionuclide labelled uPAR binding conjugate for use according to any one of embodiments 1 to 8, wherein the chelating agent is selected from the group consisting of DOTA, DOTAM, NOTA, NODAGA, SARCOPHAGINE, CB-TE2A, or derivatives thereof.

Embodiment 10, the radionuclide labelled uPAR binding conjugate for use according to any one of embodiments 1 to 9, wherein the chelating agent is selected from the group consisting of DOTA, DOTAM, SARCOPHAGINE,

Embodiment 11, the radionuclide labelled uPAR binding conjugate for use according to any one of embodiments 1 to 10, wherein the chelating agent is DOTA.

Embodiment 12, the radionuclide labelled uPAR binding conjugate for use according to any one of embodiments 1 to 11, wherein the radionuclide labelled peptide conjugate has the formula:

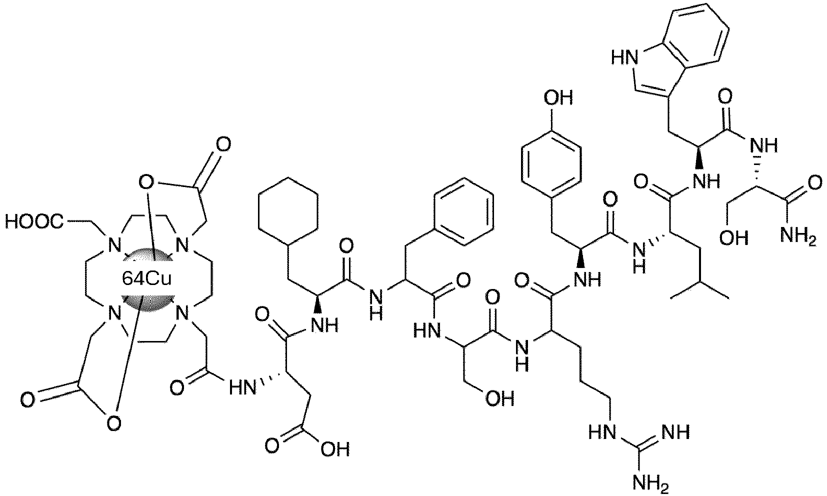

Embodiment 13, the radionuclide labelled uPAR binding conjugate for use according to any one of embodiments 1 to 12, wherein said cancer is selected from the group consisting of breast cancer, Brain cancer (in particular high grade gliomas, glioblastoma, and meningioma), NSCLC (non-small cell lung cancer), SCLC (small cell lung cancer), Neuroendocrine tumors, Head and neck cancer, HNSCC (Head and Neck squamous cell carcinoma), pancreas cancer, liver cancer, prostate cancer, CRC (Colorectal cancer), urinary bladder cancer, ovarian cancer, cervical cancer, melanoma, esophageal cancer, and gastric cancer.

Embodiment 14, the radionuclide labelled uPAR binding conjugate for use according to any one of embodiments 1 to 13, wherein said cancer is selected from the group consisting of breast cancer, NSCLC, Neuroendocrine tumors, HNSCC, pancreas cancer, prostate cancer, CRC, glioblastoma, urinary bladder cancer, and gastric cancer.

Embodiment 15, the radionuclide labelled uPAR binding conjugate for use according to any one of embodiments 1 to 14, wherein said cancer is selected from the group consisting of glioblastoma.

Embodiment 16, the radionuclide labelled uPAR binding conjugate for use according to any one of embodiments 1 to 15, wherein said radionuclide labelled uPAR binding conjugate is administered in a cumulative dose providing a calculated radiation dose to the cancer of less than 50 Gy, preferably less than 30Gy.

Embodiment 17, the radionuclide labelled uPAR binding conjugate for use according to any one of embodiments 1 to 16, wherein the cumulative dose administered systemically preferably by the intravenous route to a human patient is in the range of 40 GBq to 200 GBq, such as 40GBq to 163GBq, preferably 50GBq to 112GBq.

Embodiment 18, the radionuclide labelled uPAR binding conjugate for use according to any one of embodiments 1 to 16, wherein the radionuclide labelled peptide conjugate is administered locally.

Embodiment 19, the radionuclide labelled uPAR binding conjugate for use according to embodiment 18, wherein the radionuclide labelled peptide conjugate is administered locally to the brain or bladder.

Embodiment 20, the radionuclide labelled uPAR binding conjugate for use according to any one of embodiments 18 to 19, wherein a cumulative dose of up to 335GBq installed for 15 min to 2 h or up to 1,340GBq installed for 15 min or less, preferably in the range of 50-335GBq installed for 15min to 2 h or 200-1,340GBq installed for 15 min or less, most preferred in the range of 100-335GBq installed for 15min to 2 h or 400-1,340GBq if installed for 15 min or less is administered locally to the urinary bladder.

Embodiment 21, the radionuclide labelled uPAR binding conjugate for use according to any one of embodiments 18 to 20, wherein a cumulative dose of up to 335GBq installed for 15min to 1 h or up to 1,340GBq installed for 5 min to 15 min, preferably in the range of 50-335GBq installed for up to 1 h or 200-1,340GBq installed for 5 min to 15 min, most preferred in the range of 100-335GBq installed for up to 1 h or 400-1,340GBq if installed for 5 min to 15 min is administered locally to the urinary bladder by bladder installation.

Embodiment 22, the radionuclide labelled uPAR binding conjugate for use according to any one of embodiments 20 to 21, wherein said cumulative dose is administered in single doses of 2 GBq to 10 GBq.

Embodiment 23, a method of treatment of a uPAR expressing cancer disease by administering to a patient a radionuclide labeled uPAR binding conjugate, wherein sad radionuclide labeled uPAR binding conjugate comprises:
a) A uPAR binding moiety
b) A chelating agent suitable for binding radiometals
c) The radionuclide 64Cu
And wherein the peptide is coupled to 64Cu by the chelating agent.

Embodiment 24, the method of treatment according to embodiment 23, wherein said uPAR binding moiety is a uPAR binding peptide

Embodiment 25, the method of treatment according to any one of embodiments 23 to 24, wherein said radionuclide labelled uPAR binding conjugate consists of:
a) A uPAR binding moiety, preferably a uPAR binding peptide
b) A chelating agent suitable for binding radiometals
c) The radionuclide 64Cu
And wherein the peptide is coupled to 64Cu by the chelating agent.

Embodiment 26, the method of treatment according to any one of embodiment 24 to 25, wherein the uPAR binding peptide comprises the sequence **([beta]-cyclohexyl-L-alanine)-(Phe)**-X-X-X, wherein x designate any amino acid.

Embodiment 27, the method of treatment according to any one of embodiments 24 to 26, wherein the uPAR binding peptide comprises the sequence x-Cyclohexyl-(L)alanine - Phe-x-x-x-Leu-Trp-x, wherein x designates any amino acid.

Embodiment 28, the method of treatment according to any one of embodiments 24 to 27, wherein the uPAR binding peptide is a monomeric peptide or a multimeric peptide wherein the one or more peptide constituent independently has a length of 5 to 25 amino acids, preferably 7 to 15 amino acids, more preferred 8 to 12 amino acids, most preferred 9 amino acids.

Embodiment 29, the method of treatment according to embodiment 28, wherein the one or more peptide constituent is independently selected from the group consisting of:
(D-Asp)-([beta]-cyclohexyl-L-alanine)-(Phe)-(D-Ser)-(D-Arg)-(Tyr)-(Leu)-(Trp)-(Ser),
(Ser)-(Leu)-([beta]-cyclohexyl-L-alanine)-(Phe)-(D-Ser)-(Gln)-(Tyr)(Leu)-(Trp)-(Ser),
(D-Glu)-([beta]-cyclohexyl-L-alanine)-(Phe)-(D-Ser)-(D-Tyr)-(Tyr)-(Leu)-(Trp)-(Ser),
(Asp)-([beta]-cyclohexyl-L-alanine)-(Phe)-(D-Ser)-(D-Arg)-(Tyr)-(Leu)-(Trp)-(Ser),
(Asp)-([beta]-cyclohexyl-L-alanine)-(Phe)-(Ser)-(D-Arg)-(Tyr)-Leu)-(Trp)-(Ser),
(D-Asp)-([beta]-cyclohexyl-L-alanine)-(Phe)-(Ser)-(D-Arg)-(Tyr)-Leu)-(Trp)-(Ser),
(D-Thr)-([beta]-cyclohexyl-L-alanine)-(Phe)-(D-Ser)-(D-Arg)-(Tyr)-(Leu)-(Trp)-(Ser),
(D-Asp)-([beta]-cyclohexyl-L-alanine)-(Phe)-(D-Ser)-(D-Arg)-(Tyr)-(Leu)-([beta]-2-naphthyl-L-alanine)-(Ser),
(Asp)-([beta]-cyclohexyl-L-alanine)-(Phe)-(D-Ser)-(Arg)-(Tyr)-(Leu)-(Trp)-(Ser),
(Asp)-([beta]-cyclohexyl-L-alanine)-(Phe)-(D-Ser)-(D-Arg)-(Tyr)-(Leu)([beta]-1-naphthyl-L-alanine)-(Ser),
(D-Glu)-([beta]-cyclohexyl-L-alanine)-(Phe)-(D-Ser)-(Tyr)-(Tyr)-(Leu)-(Trp)-(Ser),
(Asp)-([beta]-cyclohexyl-L-alanine)-(Phe)-(D-Ser)-(D-Arg)-(Leu)-(Leu)-(Trp)-(D-His),
(Asp)-([beta]-cyclohexyl-L-alanine)-(Phe)-(D-Ser)-(D-Arg)-([beta]-cyclohexyl-L-alanine)-(Leu)-(Trp)-(!!e),
(Asp)-([beta]-cyclohexyl-L-alanine)-(Phe)-(D-Ser)-(D-Arg)-(Tyr)-(Leu)([beta]-1-naphthyl-L-alanine)-(D-His),
(Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine), (Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine),and
(Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(lle).

Embodiment 30, the method of treatment according to any one of embodiments 24 to 29, wherein the uPAR binding peptide is (D-Asp)-([beta]-cyclohexyl-L-alanine)-(Phe)-(D-Ser)-(D-Arg)-(Tyr)-(Leu)-(Trp)-(Ser)

Embodiment 31, the method of treatment according to any one of embodiments 23 to 30, wherein the chelating agent is selected from the group consisting of DOTA, DOTAM, NOTA, NODAGA, SARCOPHAGINE, CB-TE2A, or derivatives thereof.

Embodiment 32, the method of treatment according to any one of embodiments 23 to 30, wherein the chelating agent is selected from the group consisting of DOTA, DOTAM, SARCOPHAGINE,

Embodiment 33, the method of treatment according to any one of embodiments 23 to 32, wherein the chelating agent is DOTA.

Embodiment 34, the method of treatment according to any one of embodiments 23 to 33, wherein the radionuclide labelled peptide conjugate has the formula:

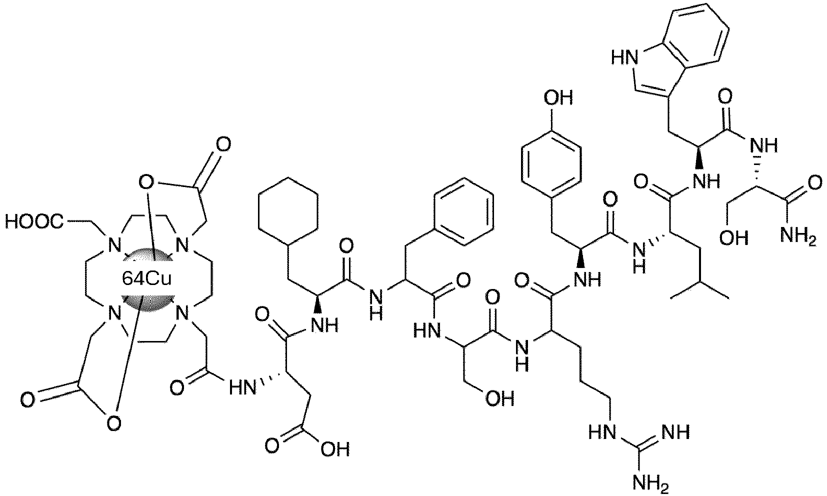

Embodiment 35, the method of treatment according to any one of embodiments 23 to 34, wherein said cancer is selected from the group consisting of breast cancer, Brain cancer, in particular high grade gliomas, glioblastoma, NSCLC(non-small cell lung cancer), SCLC (small cell lung cancer), Neuroendocrine tumors, Head and neck cancer, HNSCC (Head and Neck squamous cell carcinoma), pancreas cancer, prostate cancer, CRC (Colorectal cancer), and gastric cancer.

Embodiment 36, the method of treatment according to any one of embodiments 23 to 35, wherein said cancer is selected from the group consisting of breast cancer, NSCLC, Neuroendocrine tumors, HNSCC, pancreas cancer, prostate cancer, CRC, glioblastoma, and gastric cancer.

Embodiment 37, the method of treatment according to any one of embodiments 23 to 36, wherein said cancer is selected from the group consisting of glioblastoma.

Embodiment 38, the method of treatment according to any one of embodiments 23 to 37, wherein said radionuclide labelled uPAR binding conjugate is administered in a cumulative dose providing a calculated radiation dose to the cancer of less than 50 Gy, preferably less than 30Gy.

Embodiment 39, the method of treatment according to any one of embodiments 23 to 38, wherein the cumulative dose administered systemically preferably by the intravenous route to a human patient is in the range of 40 GBq to 200 GBq, such as 40GBq to 163GBq, preferably 50GBq to 112GBq.

Embodiment 40, the method of treatment according to any one of embodiments 23 to 39, wherein the radionuclide labelled peptide conjugate is administered locally.

Embodiment 41, the method of treatment according to any one of embodiments 23 to 40, wherein the radionuclide labelled peptide conjugate is administered locally to the brain or bladder.

Embodiment 42, the method of treatment according to any one of embodiments 40 to 41, wherein a cumulative dose of up to 335GBq installed for 15min to 2 h or up to 1,340GBq installed for 15 min or less, preferably in the range of 50-335GBq installed for 15min to 2 h or 200-1,340GBq installed for 15 min or less, most preferred in the range of 100-335GBq installed for 15min to 2 h or 400-1,340GBq if installed for 15 min or less is administered locally to the urinary bladder.

Embodiment 43, the method of treatment according to any one of embodiments 40 to 42, wherein a cumulative dose of up to 335GBq installed for 15 min to 1 h or up to 1,340GBq installed for 5 min to 15 min, preferably in the range of 50-335GBq installed 15 min to 1 h or 200-1,340GBq installed 5 min to 15 min, most preferred in the range of 100-335GBq installed for 15 min to 1 h or 400-1,340GBq if installed 5 min to 15 min is administered locally to the urinary bladder by bladder installation.

Embodiment 44, the method of treatment according to any one of embodiments 42 to 43, wherein said cumulative dose is administered in single doses of 2 GBq to 10 GBq.

Embodiment 54, a pharmaceutical formulation comprising a radionuclide labeled uPAR binding peptide conjugate comprising:
a) A uPAR binding moiety
b) A chelating agent suitable for binding radiometals
c) The radionuclide 64Cu
And wherein the peptide is coupled to 64Cu by the chelating agent.

### EXAMPLES

### Example 1: Radiolabeling

### 64Cu labelling of DOTA-AE105 conjugate

A radionuclide labelled uPAR binding peptide conjugate according to the present invention was prepared by 64Cu labelling of DOTA-AE105 conjugate.

The compound was labeled with suitable molar activity, e.g. 25 MBq/nmol and 322.4 MBq/nmol to generate stock solutions from which relevant doses such as 10 MBq 19 MBq, 30 MBq, 37 MBq, 60 MBq, 111 MBq and 130 MBq could be intravenously administered.

Compound(s) was labeled with a specific activity of 10 MBq/nmol (Biodistribution studies, Cu64), 322.4 MBq/nmol (Efficacy studies Cu64).

*When labeling with 64Cu, the dried 64Cu was dissolved in Trace select water to an activity concentration between 2-30 GBq*/*mL.*
- The desired activity was transferred to an Eppendorf tube, and the activity was measured.
- Labeling buffer (for low specific activity:0.25 M NaOAc, for High specific activity: 3 M NaOAc w. 50 mg/mL ascorbic acid) was added in ratio 1:1 or to obtain a reaction volume between 80-200.
- The pH was measured by indicator paper. Estimated pH 5.0-5.5.
- The amount of compound was added to reach the desired molar activity.
- Reaction mixture was stirred at 80°C, 600 RPM for up to 15 min.
- The product may be purified depending on the degree of incorporation.
- The final product was formulated in formulation buffer (20 mg/mL ascorbic acid, 1 mg/mL Tween-20, 5% EtOH in Sterile NaCl for injection) to the desired activity concentration and compounds concentration.
- The radiochemical purity (RCP) of the final product (EOS) was analyzed by radio-HPLC and radio-TLC.

### Example 2: Biodistribution of 64Cu-DOTA-AE105

The biodistribution of 64Cu-DOTA-AE105 was evaluated in female NMRI nude mice bearing subcutaneous U87 MG tumors.

Three mice were included for *in vivo* and *ex vivo* biodistribution (Group A) on study day -1. The mean (±SEM) tumor volume at inclusion was 245.3 ± 48.5 mm³ (range: 181.1-340.3 mm³) and the mean (±SEM) inclusion weight was 27.9 ± 1.4 g (range: 26.4- 30.6 g).

Each mouse received a single dose of 10 MBq 64Cu-DOTA-AE105 and underwent PET/CT imaging 0.5H, 2H and 24h post-dosing. ex *vivo* biodistribution was evaluated after the last imaging at the 24H time point.

Representative axial and maximum intensity projection (MIP) images with scale bar 0-20 %ID/g of one animal from group A are shown in Figure 1A.

The results of *in vivo* biodistribution of 10 MBq Cu-64-DOTA-AE105 is showed in figure 1B and from the figure it can be appreciated that tumor uptake increased within 24 hours from 3.2 %ID/g at 0.5 hours to 5.4 %ID/g after 24 hours. Conversely, it was observed that the liver uptake decreased over time from 12.9 %ID/g at 0.5 hours to 6.3 %ID/g after 24 hours.

Results from ex vivo biodistribution is shown in figure 1C. From figure 1C it can be appreciated that 24 hours post-dosing, the liver displayed the highest *ex vivo* uptake, with a mean uptake of 7.4 %ID/g, while the tumor exhibited the second-highest uptake, with a mean of 6.7 %ID/g.

### Example 3: Therapeutic effect of 64Cu-DOTA-AE105 in glioblastoma bearing xenograft mice

The treatment efficacy of 64Cu-DOTA-AE105 was tested in female NMRI nude mice bearing subcutaneous U87 MG tumors.

The U87 MG tumor cell line is a human glioblastoma cell line, and tumors were established subcutaneously in the flank region of the female NMRI nude mice. Ten animals were included and stratified into two groups based on tumor volume and body weight (n=5/group) on study day -1.

The mean (±SEM) tumor volume at inclusion was 76.5 ± 8.2 mm³ (range: 50.0-131.0 mm³) and the mean (±SEM) inclusion weight was 30.9 ± 0.7 g (range: 27.9- 33.7 g). No significant difference was observed for tumor volume or body weight between the groups (Student's t-test, p=0.94).

Animals received either vehicle (untreated control) or 130 MBq 64Cu-DOTA-AE105 at study day 0.

All mice were pre-treated with diet-gel five days prior to dosing and 5 days post dosing to prevent significant weight loss. Tumors and body weights were measured twice weekly for the duration of the study. Animals were taken down due to humane endpoint for tumor volume (≥ 1500mm³). The last observation for euthanized animals was carried forward until less than 60% of the animals were alive within the group.

The results are shown in figure 2A from which it can be appreciated that a single dose of 130 MBq 64Cu-DOTA-AE105 significantly delayed tumor growth up to 17 days post dosing in the xenograft mice compared to vehicle control group.

Tumor volumes were compared between the two groups at the latest timepoint where 60% or more animals were alive within each group. The results are shown in figure 2B. Figure 2B shows tumor volume comparison between treatment with 64Cu-DOTA-AE105 and Vehicle control group at study day 13. The treatment with 130 MBq 64Cu-DOTA-AE105 led to a significant reduction in tumor growth and end tumor volume compared to animal receiving Vehicle (Unpaired t-test, p=0.0139). Additionally, relative animal body weight was unaffected on day 5 post-dosing.

Figure 2C shows Kaplan-meier survival curves. It can be appreciated that the median survival for animals treated with 64Cu-DOTA-AE105 was 27 days, compared to 20 days for individuals in the Vehicle group, yielding a significant increase in survival for the treated group (Mantel Cox's test, P= 0.0164).

### Example 4: Therapeutic effect of 67Cu-DOTA-AE105 in glioblastoma bearing xenograft mice

For benchmarking of the therapeutic efficacy of 64Cu-DOTA-AE105 in cancer therapy, the efficacy was compared to that of 67Cu labelled DOTA-AE105 conjugate (64Cu-DOTA-AE105). The results of example 3 was compared to the result of the following experiment.

The treatment efficacy of 67Cu-DOTA-AE105 was evaluated in female NMRI nude mice bearing subcutaneous U87 MG tumors. The U87 MG tumor cell line is a human glioblastoma cell line.

Animals were stratified into four groups (A, B, C, and D) based on tumor volume and body weight on study day -2. The mean inclusion volume was 131.2 ± 8.6 mm³ (range 66.0 - 258.3 mm³), while the inclusion weight was 29.5 ± 04 g (range 24.2 - 32.9 g). No significant difference in tumor volume or animal body weight was observed between the groups (one-way ANOVA, p>0.9 and p=0.3, respectively).

Animals were dosed with either vehicle (group A) or 67Cu-DOTA-AE105 (group B, C and D) at different activity levels on study day 0. Animals in group B received 29.6 ± 0.1 MBq, animals in group C received 29.6 ± 0.1 MBq, and animals in group D received 59.5 ± 0.2 MBq. At study day 14 animals in group C received an additional dosing with 30.1 ± 0.1 MBq. Data is presented as dosed measured - residual dose. No decay correction has been applied.

Tumor volume and body weight was monitored twice a week after the first dosing. The efficacy was evaluated over a period of 35 days. The mean tumor volumes for animals included in the study is presented in figure 3A. As can be observed in figure 3A treatment with 60MBq 67Cu-DOTA-AE105 inhibited tumor growth up to 13 days post dosing. Differences in tumor volume were evaluated at the latest timepoint where minimum 50% of animals were alive within each of the compared groups.

On study day 17 all treatment groups had significantly smaller tumor volumes when compared to the vehicle control group (Dunnett's; A vs. B; p<0.03, A vs. C: p=0.02 and A vs. D: p<0.0001).

Animals were euthanized according to humane endpoints, which was reached when tumor sizes > 1500 mm³. Kaplan-Meier curves of survival data is presented in figure 3B. The median survival time for animals in the vehicle control group A was 17 days while animals in group B, C, and D had a median survival time of 21, 21, and 29 days, respectively.

The body weight did not differ between the groups during the study, indicating that the drug was safe and well tolerated.

67Cu decays by β⁻(beta minus)-decay and has a half-life of 2.6 days. 67Cu is well known for therapeutic applications such as targeted radiotherapy. 64Cu on the contrary is well known as an imaging agent and not for therapy due to the limited β⁻(beta minus)-decay. Theoretically, providing 2.5-fold MBq of 64Cu would provide an equal dose of β⁻(beta minus)-irradiation as a given dose of 67Cu. Thus, figure 2 showing data from glioblastoma bearing xenograft mice treated with 130 MBq 64Cu-DOTA-AE105 theoretically equaling a dose of 52 MBq 67 Cu (130 divided by 2.5 = 52) is expected to show a similar or reduced therapeutic effect compared to the results shown in figure 3 showing data from glioblastoma bearing xenograft mice treated with 60MBq 67Cu-DOTA-AE105. Surprisingly, it can be appreciated that treatment with 64Cu-DOTA-AE105 provided a significantly delayed tumor growth up to 17 days post dosing as compared to 13 days observed for treatment with 60MBq 67Cu-DOTA-AE105 in the same tumor model. Thus, surprisingly the dose of 64Cu labelled DOTA-AE105 compound resulted in a better therapeutic effect than treatment with 60MBq 67Cu-DOTA-AE105.

### Example 5: Dose-response of 64Cu-DOTA-AE105 in glioblastoma bearing xenograft mice

The dose dependent efficacy of 64Cu-DOTA-AE105 was tested in female NMRI nude mice bearing subcutaneous U87 MG tumors (see figure 4). The U87 MG tumor cell line is a human glioblastoma cell line.

Animals were stratified into five groups (A, B, C, D, and E) based on tumor volume and body weight on study day -2. The mean inclusion volume was 98.3 ± 8.0 mm3 (range: 39.3-191.0 mm3 and the mean (±SEM) inclusion weight was 29.6 ± 0.4 g (range: 24.8-32.7 g). No significant difference was observed for tumor volume or body weight between the groups (One-way ANOVA, p=0.9953 and p=0.9624, respectively).

Animals were dosed with either vehicle (group A) or 64Cu-DOTA-AE105 (group B, C, D, and E) at different activity levels on study day 0. Animals in group B received 111 MBq, animals in group C received 37 MBq, animals in group D received 19 MBq, and animals in group E received 37 MBq. On study day 7 and 14 animals in group E received an additional dosing with 37 MBq.

Tumor volume and body weight was monitored twice a week after the first dosing. The efficacy was evaluated over a period of 27 days. The mean tumor volumes for animals included in the study is presented in figure 4. Differences in tumor volume were evaluated at the latest timepoint where minimum 60% of animals were alive within each of the compared groups. Figure 4B shows tumor volume comparison on study day 17 (the last day where 60% or more animals were alive within all groups). The effect of 19, 37, and 111 MBq Cu-64-DOTA-AE105 (Group B-D) and the effect of fractionated dosing of 37 MBq Cu-64-DOTA-AE105 (Group E) were tested against Vehicle (Group A) by One-way ANOVA with Dunnett's multiple comparison (summary of the statistical analysis is shown in the table below, statistical significance is highlighted in bold). A significant effect was observed following treatment with 111 MBq Cu-64-DOTA-AE105 compared to Vehicle and 37 MBq Cu-64-DOTA-AE105 as fractionated dosing. Bars represent mean ± SEM. N=3-5/group.

| **Group comparison** | **Statistical test** | **Study day** | **P value** |
|---|---|---|---|
| A - Vehicle vs. B - 64Cu-AE105 (111MBq) | One-way ANOVA with Dunnett's correction for multiple comparison | 17 | **0.0009** |
| A - Vehicle vs. C - 64Cu-AE105 (37MBq) | | | 0.4240 |
| A - Vehicle vs. D - 64Cu-AE105 (19MBq) | | | 0.5186 |
| A - Vehicle vs. E - 64Cu-AE105 (37MBq 3xQW) | | | **0.0089** |

Animals were euthanized according to humane endpoints, which was reached when tumor sizes > 1500 mm³. Kaplan-Meier curves of survival data is presented in figure 5. The survival analysis, illustrated in figure 5, shows a median survival of 17 days for Vehicle and group C (37 MBq Cu-64-DOTA-AE105), a median survival of 18.5 for group D (19 MBq Cu-64-DOTA-AE105) and a median survival time of 24 days for group B and E (111 MBq and 37 (3xQW) Cu-64-DOTA-AE105).

Summary of statistical analysis of pairwise comparison of the survival is shown in the table below. All treatment groups were compared to the vehicle group by Log-rank (Mantel-Cox) test with Bonferroni correction for multiple comparison. The Bonferroni corrected threshold, used for each individual comparison, is the familywise significance level (0.05) divided by the number of comparisons performed (4). Statistical significance is highlighted in Bold.

| **Group comparison** | **Statistical test** | **Bonferroni-corrected threshold (α_{f})** | **P value** |
|---|---|---|---|
| A - Vehicle vs. B - 64Cu-AE105 (111MBq) | Log-rank (Mantel-Cox) test with Bonferroni correction for multiple comparison | 0.05/4= 0.0125 | **0.0062** |
| A - Vehicle vs. C - 64Cu-AE105 (37MBq) | | | 0.3533 |
| A - Vehicle vs. D - 64Cu-AE105 (19MBq) | | | 0.0944 |
| A - Vehicle vs. E - 64Cu-AE105 (37MBq 3xQW) | | | **0.0100** |

The body weight did not differ between the groups during the study, indicating that the drug was safe and well tolerated.

As can be observed in figure 4, treatment with 64Cu-DOTA-AE105 provided a surprisingly effective reduction of tumor growth. Treatment with a single dose of 111 MBq 64Cu-DOTA-AE105 (group B) significantly and almost entirely inhibited tumor growth up to 17 days post dosing thereby confirming the treatment efficacy observed in figure 2A even with a slightly lower dose, i.e. 111 MBq compared to 130MBq. Even more striking, it was observed that treatment with three doses of 37 MBq 64Cu-DOTA-AE105 administered once a week (group E, figure 4) delayed tumor growth up to 17 days post first dosing at almost the same efficacy as treatment with a single dose of 111MBq 64Cu-DOTA-AE105. Thus, 64Cu-DOTA-AE105 showed significant therapeutic efficacy compared to untreated control group even at very low doses, equaling (i.e., delivering same radiation dose in Gy to the tumor) approximately 15 MBq 67Cu at which doses 67Cu labeled compound, based on available data, e.g. in figure 3, would not have any therapeutic effect.

### Example 6: Therapeutic effect of 64Cu-DOTA-AE105 in non-small cell lung cancer (NSCLC) bearing xenograft mice

The treatment efficacy of 64Cu-DOTA-AE105 was tested in female NMRI nude mice bearing subcutaneous NCI-H1993 tumors.

The NCI-H1993 tumor cell line is a human non-small cell lung cancer (NSCLC) cell line, and tumors were established subcutaneously in the flank region of the female NMRI nude mice. Ten animals were included and stratified into two groups based on tumor volume and body weight (n=5/group).

The mean (±SEM) tumor volume at inclusion was 79.1 ± 6.9 mm³ (range: 47.1-111.7 mm³) and the mean (±SEM) inclusion weight was 31.2 ± 0.6 g (range: 27.5- 34.8 g). No significant difference was observed for tumor volume or body weight between the groups (Student's t-test p=0.98 and p=0.33, respectively).

Animals received either vehicle (untreated control) or 150 MBq Cu-64-DOTA-AE105 at study day 0.

All mice were pre-treated with diet-gel five days prior to dosing and 5 days post dosing to prevent significant weight loss. Tumors and body weights were measured twice weekly for the duration of the study. Animals were taken down due to humane endpoint for tumor volume (≥ 1500mm³). The last observation for euthanized animals was carried forward until less than 60% of the animals were alive within the group.

The results are shown in figure 6 from which it can be appreciated that a single dose of 150 MBq 64Cu-DOTA-AE105 significantly inhibited tumor growth for the entire duration of the study.

Tumor volumes were compared between the group treated with 64Cu-DOTA-AE105 (Group B) and Vehicle (Group C) at study day 57. The treatment with 150MBq 64Cu-DOTA-AE105 led to a significant reduction in tumor volume compared to animal receiving Vehicle (Unpaired t-test, p=0.0113).

As can be appreciated from figure 6, the 64Cu labelled uPAR binding peptide conjugate 64Cu-DOTA-AE105 stalled tumor growth for the entire duration of the study, thus providing effective treatment of human NCl-H1993 tumors.

## Claims

1. A radionuclide labelled uPAR binding conjugate for use in treatment of uPAR expressing cancer, wherein said radionuclide labelled uPAR conjugate comprises:
a) A uPAR binding moiety
b) A chelating agent suitable for binding radiometals
c) The radionuclide 64Cu
And wherein the peptide is coupled to 64Cu by the chelating agent.

2. The radionuclide labelled uPAR binding conjugate for use according to claim 1, wherein said uPAR binding moiety is a uPAR binding peptide

3. The radionuclide labelled uPAR binding conjugate for use according any one of claims 1 to 2, wherein said radionuclide labelled uPAR binding conjugate consists of
a) A uPAR binding moiety, preferably a uPAR binding peptide
b) A chelating agent suitable for binding radiometals
c) The radionuclide 64Cu
And wherein the peptide is coupled to 64Cu by the chelating agent.

4. The radionuclide labelled uPAR binding conjugate for use according to any one of claims 2 to 3, wherein the uPAR binding peptide comprises the sequence **([beta]-cyclohexyl-L-alanine)-(Phe)**-X-X-X, wherein x designates any amino acid.

5. The radionuclide labelled uPAR binding conjugate for use according to any one of claims 2 to 4, wherein the uPAR binding peptide is a monomeric peptide or a multimeric peptide wherein the one or more peptide constituent independently has a length of 5 to 25 amino acids, preferably 7 to 15 amino acids, more preferred 8 to 12 amino acids, most preferred 9 amino acids.

6. The radionuclide labelled uPAR binding conjugate for use according to claim 5, wherein the one or more peptide constituent is independently selected from the group consisting of:
(D-Asp)-([beta]-cyclohexyl-L-alanine)-(Phe)-(D-Ser)-(D-Arg)-(Tyr)-(Leu)-(Trp)-(Ser),
(Ser)-(Leu)-([beta]-cyclohexyl-L-alanine)-(Phe)-(D-Ser)-(Gln)-(Tyr)(Leu)-(Trp)-(Ser),
(D-Glu)-([beta]-cyclohexyl-L-alanine)-(Phe)-(D-Ser)-(D-Tyr)-(Tyr)-(Leu)-(Trp)-(Ser),
(Asp)-([beta]-cyclohexyl-L-alanine)-(Phe)-(D-Ser)-(D-Arg)-(Tyr)-(Leu)-(Trp)-(Ser),
(Asp)-([beta]-cyclohexyl-L-alanine)-(Phe)-(Ser)-(D-Arg)-(Tyr)-Leu)-(Trp)-(Ser),
(D-Asp)-([beta]-cyclohexyl-L-alanine)-(Phe)-(Ser)-(D-Arg)-(Tyr)-Leu)-(Trp)-(Ser),
(D-Thr)-([beta]-cyclohexyl-L-alanine)-(Phe)-(D-Ser)-(D-Arg)-(Tyr)-(Leu)-(Trp)-(Ser),
(D-Asp)-([beta]-cyclohexyl-L-alanine)-(Phe)-(D-Ser)-(D-Arg)-(Tyr)-(Leu)-([beta]-2-naphthyl-L-alanine)-(Ser),
(Asp)-([beta]-cyclohexyl-L-alanine)-(Phe)-(D-Ser)-(Arg)-(Tyr)-(Leu)-(Trp)-(Ser),
(Asp)-([beta]-cyclohexyl-L-alanine)-(Phe)-(D-Ser)-(D-Arg)-(Tyr)-(Leu)([beta]-1-naphthyl-L-alanine)-(Ser),
(D-Glu)-([beta]-cyclohexyl-L-alanine)-(Phe)-(D-Ser)-(Tyr)-(Tyr)-(Leu)-(Trp)-(Ser),
(Asp)-([beta]-cyclohexyl-L-alanine)-(Phe)-(D-Ser)-(D-Arg)-(Leu)-(Leu)-(Trp)-(D-His),
(Asp)-([beta]-cyclohexyl-L-alanine)-(Phe)-(D-Ser)-(D-Arg)-([beta]-cyclohexyl-L-alanine)-(Leu)-(Trp)-(Ile),
(Asp)-([beta]-cyclohexyl-L-alanine)-(Phe)-(D-Ser)-(D-Arg)-(Tyr)-(Leu)([beta]-1-naphthyl-L-alanine)-(D-His),
(Asp)-([beta]-cyclohexyl-L-alanine)-(Phe)-(D-Ser)-(D-Arg)-(N-(2,3-dimethoxybenzyl)glycine)-(D-Phe)-(N-(3-indolylethyl)glycine)-(N-(2-methoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-benzylglycine)-(N-(2[beta]thoxyethyl)glycine),
(Asp)-([beta]-cyclohexyl-L-alanine)-(Phe)-(D-Ser)-(D-Arg)-(N-(2,3-dimethoxybenzyl)glycine)-(D-Phe)-(N-(methylnaphthalyl)glycine)-(N-(2-methoxyethyl)glycine),and
(Asp)-([beta]-cyclohexyl-L-alanine)-(Phe)-(D-Ser)-(D-Arg)-(N-(2,3-dimethoxybenzyl)glycine)-(D-Phe)-(N-(2,3-dimethoxybenzyl)glycine)-(lle).

7. The radionuclide labelled uPAR binding conjugate for use according to any one of claims 2 to 6, wherein the uPAR binding peptide is (D-Asp)-([beta]-cyclohexyl-L-alanine)-(Phe)-(D-Ser)-(D-Arg)-(Tyr)-(Leu)-(Trp)-(Ser)

8. The radionuclide labelled uPAR binding conjugate for use according to any of the preceding claims, wherein the chelating agent is selected from the group consisting of DOTA, DOTAM, NOTA, NODAGA, SARCOPHAGINE, CB-TE2A, or derivatives thereof.

9. The radionuclide labelled uPAR binding conjugate for use according to any of the preceding claims, wherein the radionuclide labelled peptide conjugate has the formula:

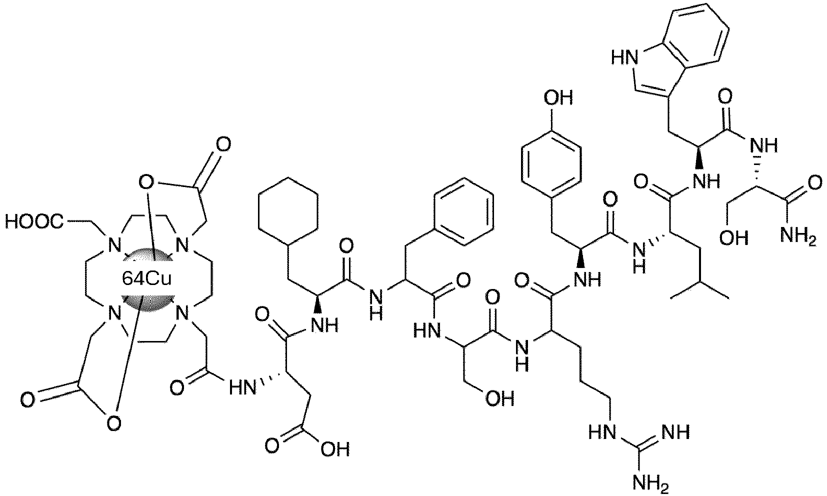

10. The radionuclide labelled uPAR binding conjugate for use according to any of the preceding claims, wherein said cancer is selected from the group consisting of breast cancer, Brain cancer (in particular high grade gliomas, glioblastoma, and meningioma), NSCLC (non-small cell lung cancer), SCLC (small cell lung cancer), Neuroendocrine tumors, Head and neck cancer, HNSCC (Head and Neck squamous cell carcinoma), pancreas cancer, liver cancer, prostate cancer, CRC (Colorectal cancer), urinary bladder cancer, ovarian cancer, cervical cancer, melanoma, esophageal cancer, and gastric cancer.

11. The radionuclide labelled uPAR binding conjugate for use according to any of the preceding claims, wherein said cancer is selected from the group consisting of glioblastoma.

12. The radionuclide labelled uPAR binding conjugate for use according to any of the preceding claims, wherein said radionuclide labelled uPAR binding conjugate is administered in a cumulative dose providing a calculated radiation dose to the cancer of less than 50 Gy, preferably less than 30Gy.

13. The radionuclide labelled uPAR binding conjugate for use according to any of the preceding claims, wherein the cumulative dose administered systemically preferably by the intravenous route to a human patient is in the range of 40 GBq to 200 GBq, such as 40GBq to 163GBq, preferably 50GBq to 112GBq.

14. The radionuclide labelled uPAR binding conjugate for use according to any of the claims 1 to 16, wherein the radionuclide labelled peptide conjugate is administered locally.

15. The radionuclide labelled uPAR binding conjugate for use according to any of the claims 18 to 19, wherein a cumulative dose of up to 335GBq installed for 15 min to 2 h or up to 1,340GBq installed for 15 min or less, preferably in the range of 50-335GBq installed for 15min to 2 h or 200-1,340GBq installed for 15 min or less, most preferred in the range of 100-335GBq installed for 15min to 2 h or 400-1,340GBq if installed for 15 min or less is administered locally to the urinary bladder.
